# EUROPÄISCHE PATENTANMELDUNG

(11) **EP 1 642 887 A1**
(43) Veröffentlichungstag der Anmeldung: **05.04.2006**
(21) Anmeldenummer: 04022273.9
(22) Anmeldetag: 18.09.2004
(51) Int. Cl.: C07C 235/10, C11D 1/62

(54) **Quaternisierte Fettsäureamidoamine**

(71) Anmelder: Cognis IP Management GmbH, 40589 Düsseldorf (DE)
(72) Erfinder: Bigorra Llosas, Joaquin, Dr., 08201 Sabadell (ES); Bonastre Gilabert, Nuria, Dr., 08210 Barbera del Vall (ES); Sanchez, Agustin, 08921 Santa Coloma De Gramenet (ES)

(57) **Zusammenfassung**

Vorgeschlagen werden kationische Tenside der Formel (I) 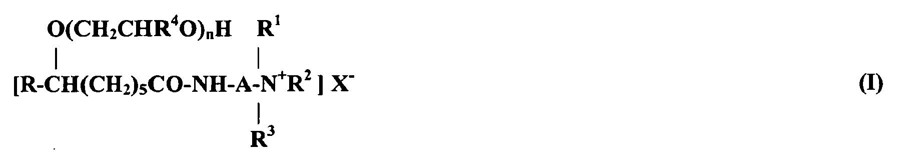 in der R für einen Alkyl- oder Alkenylrest mit 11 Kohlenstoffatomen, R¹ und R² für Wasserstoff oder Alkylreste mit 1 bis 4 Kohlenstoffatomen, R³ für einen Alkylrest mit 1 bis 4 Kohlenstoffatomen, R⁴ für Wasserstoff oder eine Methylgruppe, A für eine lineare oder verzweigte Alkylengruppe mit 2 bis 6 Kohlenstoffatomen, n für Zahlen von 1 bis 25 und X für Halogen oder Alkylsulfat steht.

## Beschreibung

### Gebiet der Erfindung

Die Erfindung befindet sich auf dem Gebiet oberflächenaktiven Mittel und betrifft neue kationische Tenside, ein Verfahren zu ihrer Herstellung sowie ihre Verwendung zur Herstellung von kosmetischen, pharmazeutischen sowie Detergentszubereitungen.

### Stand der Technik

Quaternierte Fettsäureamidoamine stellen bekannten kationische Tenside dar. In diesem Zusammenhang sei beispielsweise auf die Europäische Patentschrift **EP 1254654 B1** (Cognis) hingewiesen, aus der die Verwendung von speziellen, nicht alkoxylierten Fettsäureamidoaminen zur Herstellung von Haarbehandlungsmitteln bekannt ist. In der **EP 1314718 A1** (Cognis) sind des weiteren kationische Tenside vorgeschlagen, die erhalten werden, indem man Fettsäuren mit Diaminen kondensiert, die als Zwischenprodukte entstehenden Imidazoline zu den Fettsäureamidoaminen hydrolysiert, deren endständigen Hydroxylgruppen ethoxyliert und die Aminogruppe anschließend in bekannter Weise quaterniert. Quaternierte Fettsäureamidoamine zeichnen sich durch interessante konditionierende Eigenschaften aus, weswegen sie vor allem Anwendung in Weichspülern und Haarbehandlungsmitteln finden. Nichtsdestotrotz weisen die Produkte eine Reihe von Nachteilen auf : so sind die Produkte insbesondere in hartem Wasser nicht unbegrenzt klar löslich, die stabile Einarbeitung von bestimmten Wirkstoffen, wie beispielsweise Siliconölen und Wirkstoffen ist nicht immer gewährleistet, die biologische Abbaubarkeit ist nach den neuen Bestimmungen des Detergentiengesetzes nicht ausreichend und zudem besteht ein grundsätzlicher Bedarf an quaternierten Amidoaminen, die sowohl gegenüber synthetischen als auch natürlichen Fasern gegenüber den Produkten des Stands der Technik eine verbesserte Avivage- und Konditionierleistung aufweisen.

Die Aufgabe der vorliegenden Erfindung hat somit darin bestanden, den genannten Nachteilen abzuhelfen und neue Kationtenside auf Amidoaminbasis zur Verfügung zu stellen, die die genannten Anforderungen gleichzeitig erfüllen.

### Beschreibung der Erfindung

Gegenstand der Erfindung sind kationische Tenside der Formel (I) 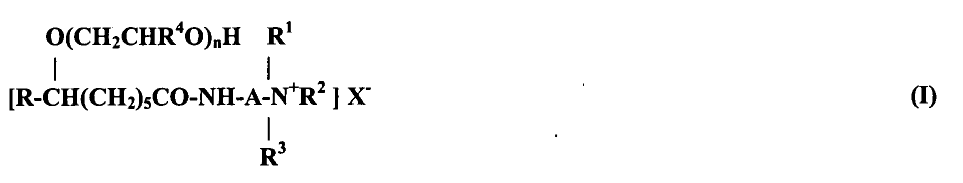 in der R für einen Alkyl- oder Alkenylrest mit 11 Kohlenstoffatomen, R¹ und R² für Wasserstoff oder Alkylreste mit 1 bis 4 Kohlenstoffatomen, R³ für einen Alkylrest mit 1 bis 4 Kohlenstoffatomen, R⁴ für Wasserstoff oder eine Methylgruppe, A für eine lineare oder verzweigte Alkylengruppe mit 2 bis 6 Kohlenstoffatomen, n für Zahlen von 1 bis 25 und X für Halogen oder Alkylsulfat steht.

In weiteren Ausführungsformen der vorliegenden Erfindung werden solche kationischen Tenside bevorzugt, bei denen
- sich die quaternierten Alkylamine der allgemeinen Formel (I) von ethoxyliertem Ricinusöl, ethoxylierter Ricinolsäure oder ethoxylierter 12-Hydroxystearinsäure ableiten;
- R¹, R² und R³ für Methylreste stehen;
- A für einen Propylenrest steht;
- n für Zahlen von 5 bis 10 steht;
- X für Chlorid oder Methylsulfat steht
sowie die Kombinationen der genannten bevorzugten Merkmale.

Überraschenderweise wurde gefunden, dass die neuen kationischen Tenside auf Amidoaminbasis nicht nur mit Wasser praktisch unbegrenzt klar mischbar ist, sondern auch gegenüber natürlichen wie synthetischen Fasern über eine verbesserte Avivage und Konditionierung verfügen. Des weiteren erlauben die Produkte auch bei längerer Lagerung unter Temperaturstress die stabile Einarbeitung beispielsweise von Siliconölen oder Wirkstoffen. Zudem besitzen die neuen Kationtenside unter den Bedingungen des geschlossenen Flaschentestes eine biologische Endabbaubarkeit von mehr als 60 %, so dass die neuen gesetzlichen Bestimmungen erfüllt werden.

### Herstellung der kationischen Tenside

Ein weiterer Gegenstand der Erfindung betrifft ein Verfahren zur Herstellung von kationischen Tensiden der Formel (I) 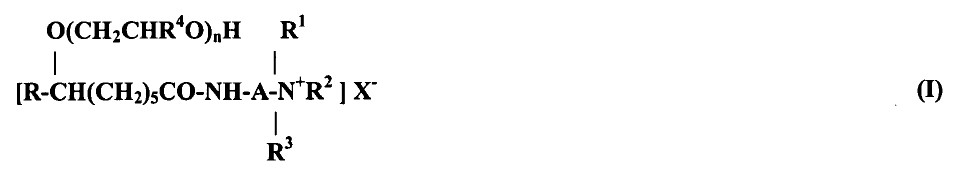 in der R für einen Alkyl- oder Alkenylrest mit 11 Kohlenstoffatomen, R¹ und R² für Wasserstoff oder Alkylreste mit 1 bis 4 Kohlenstoffatomen, R³ für einen Alkylrest mit 1 bis 4 Kohlenstoffatomen, R⁴ für Wasserstoff oder eine Methylgruppe, A für eine lineare oder verzweigte Alkylengruppe mit 2 bis 6 Kohlenstoffatomen, n für Zahlen von 1 bis 25 und X für Halogen oder Alkylsulfat steht, bei dem man (a) Anlagerungsprodukte von durchschnittlich 1 bis 25 Mol Ethylen- und/oder Propylenoxid an eine Hydroxycarbonsäure oder deren Glycerid mit einem Diamin der Formel (II) umsetzt, 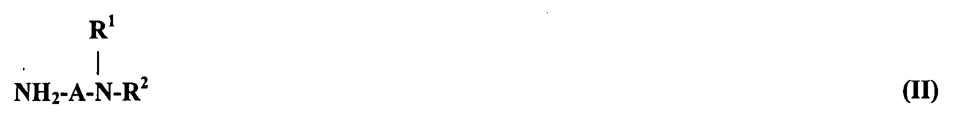 in der R¹, R² und A die oben genannten Bedeutungen haben, und (b) anschließend die so erhaltenen alkoxylierten Hydroxyfettsäureamidoamine mit einem Alkylhalogenid oder Dialkylsulfat quaterniert.

### Herstellung der Amidoamine

Die Herstellung der Amidoamine erfolgt in an sich bekannter Weise entweder durch Kondensation der entsprechenden alkoxylierten Hydroxyfettsäuren mit den Diaminen oder durch Transamidierung der alkoxylierten Triglyceride mit den Diaminen. Als Acylkomponenten kommen Anlagerungsprodukte von durchschnittlich 1 bis 25 und vorzugsweise 5 bis 10 Mol Ethylen- und/oder Propylenoxid an Ricinolsäure oder deren Härtungsprodukt, der 12-Hydroxystearinsäure sowie Mischungen beider Hydroxyfettsäuren in Frage. Alternativ können auch die entsprechenden alkoxylierten Triglyceride, also Ricinusöl, welches gegebenenfalls vollständig oder partiell gehärtet worden ist, eingesetzt werden.

In einer insgesamt bevorzugten Ausführungsform des erfindungsgemäßen Verfahrens seitzt man Anlagerungsprodukte von durchschnittlich 1 bis 25 Mol Ethylenoxid an Ricinusöl oder Ricinolsäure mit Dimethylaminopropylamin (DAPA) um und quaterniert das resultierende ethoxylierte Amidoamin anschließend mit Dimethylsulfat. Als Aminkomponente kommen beispielsweise Ethylendiamin, Propylendiamin, Butylendiamin, Pentylendiamin, Hexylendiamin, Methylaminoethylamin, Dimethylaminmethylamin, Methylaminpropylamin und insbesondere Dimethylaminopropylamin (DAPA) in Betracht. Die einfache Kondensation wie auch die Transamidierung kann in an sich bekannter Weise durchgeführt werden, d.h. in Gegenwart basischer Katalysatoren, wie beispielsweise Alkalihydroxid oder besser einem Alkalialkoholat, wie etwa Natriummethylat in Methanol oder Kalium-tert.butylat in Butanol, wobei die Einsatzmenge im Bereich von 0,5 bis 5 und vorzugsweise bei etwa 1 bis 2 Gew.-% - bezogen auf die Summe von Acyl- und Aminkomponente liegen sollte. Als Co-Katalysatoren, die auch für eine Farbstabilisierung sorgen, eignen sich Alkaliboranate, speziell Natriumborhydrid, die üblicherweise in Mengen von etwa 10 Gew.-% - bezogen auf die Katalysatoren - verwendet werden. Zur Beschleunigung der Reaktion und zur Verlagerung der Reaktion auf die Produktseite, empfiehlt es sich, eine der beiden Reaktionspartner im Überschuss einzusetzen. Dies wird in der Regel das Amin sein, da es sich dabei um die preiswertere Komponente handelt, die wegen ihres niedrigeren Siedepunktes anschließend auch leichter zu entfernen ist. Das Molare Einsatzverhältnis zwischen Acyl- und Aminkomponente beträgt in der Regel 1 : 1,1 bis 1 : 2,5 und vorzugsweise 1 : 1,5 bis 1 : 2 (wobei im Fall des Einsatzes von Triglyceriden natürlich berücksichtigt werden muss, dass diese drei Acylgruppen für die Transamidierung aufweisen). Die Reaktionstemperatur liegt vorzugsweise bei 100 bis 150 und insbesondere 120 bis 140 °C; auch höhere Temperaturen sind möglich, wirken sich aber in der Regel nachteilig auf die Farbqualität der Produkte aus. Nach einer Reaktionszeit von etwa 2 bis 3 Stunden wird nichtumgesetztes Amin, gegebenenfalls zusammen mit dem bei der Transamidierung freigesetzten Glycerin, durch Destillation im Vakuum abgetrennt.

### Quaternierung der Amidoamine

Auch die Qauternierung der zuvor hergestellten alkoxylierten Amidoamine kann in an sich bekannter Weise erfolgen. Als Alkylierungsmittel kommen Alkylhalogenide, wie beispielsweise Methylchlorid, Dialkylsulfate, vorzu8gsweise Dimethylsulfat oder auch Beznylchlorid in Betracht. Das molare Einsatzverhältnis zwischen Amidoamin und Alkylierungsmittel beträgt in der Regel 0,9 : 1 bis 1 : 1, vorzugsweise wird ein geringer Unterschuss verwendet, um sicherzustellen, dass das Endprodukt frei von nicht umgesetzten Alkylierungsmittel ist. Es hat sich als vorteilhaft erwiesen, das Alkylierungsmittel unter Rühren portionsweise, besser noch tropfenweise einzubringen und die Reaktionstemperatur, die wegen der Exothermie der Reaktion rasch ansteigt, durch Kühlung mit einem Eisbad nicht über 65 °C ansteigen zu lassen. Falls erforderlich kann das Produkt anschließend noch alkalisch eingestellt und eine Zeit bei 100 °C gerührt werden, um auch Spuren des Alkylierungsmittels zu zerstören. Aus anwendungstechnischen Gründen kann es darüber hinaus erwünscht sein, die Quaternierung nicht vollständig durchzuführen, sondern 10 bis 15 freies alkoxyliertes Amidoamin im Produkt zu belassen, da dies zu synergistischen Leistungsverbesserungen führen kann.

### Gewerbliche Anwendbarkeit

Die neuen Kationtenside weisen im vergleich zu quaternierten Amidoaminen des Stands der Technik eine verbesserte biologischen Abbaubarkeit auf, auch ihre ausgezeichneten avivierenden, konditionierenden, antistatischen und reinigenden Eigenschaften machen sie für eine Vielzahl von Anwendungen interessant. Weitere Gegenstände der vorliegenden Erfindung betreffen daher ihre Verwendung
- zur Herstellung von kosmetischen und/oder pharmazeutischen Zubereitungen, speziell Haarbehandlungsmitteln;
- zur Herstellung von Wasch-, Spül-, Reinigungs- und Avivagemitteln, vorzugsweise zur Herstellung von Entfettungsmitteln, Allzweckreinigern, Küchenreinigern, Ofenreinigern, Mitteln zur Reinigung harter Oberflächen oder Teppichreiniger;
- zur Herstellung von Mitteln zur Reinigung und Entfettung von Metallteilen, speziell Fahrzeugen und Fahrzeugteilen, beispielsweise für die Hochdruckreinigung in automatischen Waschanlagen;
- zur Herstellung von Mitteln zur Korrosionsinhibierung und Entfernung von Metalloxidbelägen;
- zur Herstellung von Mitteln zur Verminderung der statischen Aufladung von harten Oberflächen, speziell von Möbeln, Farben, Fenstern, Monitoren oder Plastikgegenständen dienen.
- sowie zur Herstellung von Mitteln zur Ausrüstung von Fasern, Garnen und Textilien.

### Kosmetische und/oder pharmazeutische Mittel

Die erfindungsgemäßen kosmetischen und/oder pharmazeutischen Mittel können weitere typische Hilfs- und Zusatzstoffe enthalten, wie beispielsweise milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, UV-Lichtschutzfaktoren, Feuchthaltemittel, biogene Wirkstoffe, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten.

### Tenside

Als oberflächenaktive Stoffe können anionische, nichtionische, kationische und/oder amphotere bzw. zwitterionische Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Alkylethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃₋Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol® CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol® OE), Ringöfföffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Emulgatoren

Als Emulgatoren kommen beispielsweise nichtionogene Tenside aus mindestens einer der folgenden Gruppen in Frage:
- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
- Mono-, Di- und Trialkylphosphate sowie Mono-, Di- und/oder Tri-PEGalkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich oder Cosmedia® SP von Cognis;
- Polyalkylenglycole sowie
- Glycerincarbonat.

### • Alkoxylate

Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18-}Fettsäuremono- und -diester von Anlagerungsprodukten von Ethylenoxid an Glycerin sind als Rückfettungsmittel für kosmetische Zubereitungen bekannt.

### • Alkyl- und/oder Alkenyloligoglykoside

Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

### • Partialglyceride

Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

### • Sorbitanester

Als Sorbitanester kommen Sorbitanmonoisostearat, Sorbitansesquiisostearat, Sorbitandiisostearat, Sorbitantriisostearat, Sorbitanmonooleat, Sorbitansesquioleat, Sorbitandioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesquitartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

### • Polyglycerinester

Typische Beispiele für geeignete Polyglycerinester sind Polyglyceryl-2 Dipolyhydroxystearate (Dehymuls® PGPH), Polyglycerin-3-Diisostearate (Lameform® TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

### • Anionische Emulgatoren

Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

### • Amphotere und kationische Emulgatoren

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### Fette und Wachse

Typische Beispiele für Fette sind Glyceride, d.h. feste oder flüssige pflanzliche oder tierische Produkte, die im wesentlichen aus gemischten Glycerinestern höherer Fettsäuren bestehen, als Wachse kommen u.a. natürliche Wachse, wie z.B. Candelillawachs, Camaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone® Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

### Stabilisatoren

Als Stabilisatoren können Metallsalze von Fettsäuren, wie z.B. Magnesium-, Aluminium- und/oder Zinkstearat bzw. -ricinoleat eingesetzt werden.

### Polymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide sowie deren vernetzte wasserlöslichen Polymere, kationische Chitinderivate wie beispielsweise quatemiertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmethacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

### Siliconverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

### UV-Lichtschutzfaktoren

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. Üblicherweise sind die UV-Lichtschutzfaktoren in Mengen von 0,1 bis 5 und vorzugsweise 0,2 bis 1 Gew.-% zugegen. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylbenzyliden)campher beschrieben;
- 4-Aminobenzoesäurederivate, vorzugsweise 4-(Dimethylamino)benzoesäure-2-ethylhexylester, 4-(Dimethylamino)benzoesäure-2-octylester und 4-(Dimethylamino)benzoe-säureamylester;
- Ester der Zimtsäure, vorzugsweise 4-Methoxyzimtsäure-2-ethylhexylester, 4-Methoxy-zimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-iso-propylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Triazon oder Dioctyl Butamido Triazone (Uvasorb® HEB);
- Propan-1,3-dione, wie z.B. 1-(4-tert.Butylphenyl)-3-(4'methoxyphenyl)propan-1,3-dion;
- Ketotricyclo(5.2.1.0)decan-Derivate.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- 1H-Benzimidazole-4,6-Disulfonic Acid, 2,2'-(1,4-Phenylene)Bis-, Disodium Salt (Neo Heliopan® AP)
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bomyliden)sulfonsäure und deren Salze.

Als typische UV-A-Filter kommen insbesondere Derivate des Benzoylmethans in Frage, wie beispielsweise 1-(4`-tert.Butylphenyl)-3-(4`-methoxyphenyl)propan-1,3-dion, 4-tert.-Butyl-4'-methoxydibenzoylmethan (Parsol® 1789), 2-(4-Diethylamino-2-hydroxybenzoyl)-benzoic acid hexylester (Uvinul® A Plus), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans,, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex® T2000, Eusolex® T, Eusolex® T-ECO, Eusolex® T-S, Eusolex® T-Aqua, Eusolex® T-45D (alle Merck), Uvinul TiO₂ (BASF). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid wie z.B. Z-COTE® oder Z-COTE HP1® verwendet.

### Feuchthaltemittel

Feuchthaltemittel dienen zur weiteren Optimierung der sensorischen Eigenschaften der Zusammensetzung sowie zur Feuchtigkeitsregulierung der Haut. Gleichzeitig wird die Kältestabilität der erfindungsgemäßen Zubereitungen, insbesondere im Falle von Emulsionen, erhöht. Die Feuchthaltemittel sind üblicherweise in einer Menge von 0,1 bis 15 Gew.-%, vorzugsweise 1 bis 10 Gew.-%, und insbesondere 5 bis 10 Gew.-% enthalten.

Erfindungsgemäß geeignet sind u.a. Aminosäuren, Pyrrolidoncarbonsäure, Milchsäure und deren Salze, Lactitol, Harnstoff und Harnstoffderivate, Harnsäure, Glucosamin, Kreatinin, Spaltprodukte des Kollagens, Chitosan oder Chitosansalze/-derivate, und insbesondere Polyole und Polyolderivate (z. B. Glycerin, Diglycerin, Triglycerin, Ethylenglycol, Propylenglycol, Butylenglycol, Erythrit, 1,2,6-Hexantriol, Polyethylenglycole wie PEG-4, PEG-6, PEG-7, PEG-8, PEG-9, PEG-10, PEG-12, PEG-14, PEG-16, PEG-18, PEG-20), Zucker und Zuckerderivate (u.a. Fructose, Glucose, Maltose, Maltitol, Mannit, Inosit, Sorbit, Sorbitylsilandiol, Sucrose, Trehalose, Xylose, Xylit, Glucuronsäure und deren Salze), ethoxyliertes Sorbit (Sorbeth-6, Sorbeth-20, Sorbeth-30, Sorbeth-40), Honig und gehärteter Honig, gehärtete Stärkehydrolysate sowie Mischungen aus gehärtetem Weizenprotein und PEG-20-Acetatcopolymer. Erfindungsgemäß bevorzugt geeignet als Feuchthaltemittel sind Glycerin, Diglycerin, Triglycerin und Butylenglycol.

### Biogene Wirkstoffe und Antioxidantien

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen.

Antioxidantien unterbrechen die photochemische Reaktionskette, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Deodorantien und keirnhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

### • Keimhemmende Mittel

Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4-dichlorphenyl)harnstoff, 2,4,4'-Trichlor-2'-hydroxy-diphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

### • Enzyminhibitoren

Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen® CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

### • Geruchsabsorber

Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

### • Antitranspirantien

Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
- adstringierende Wirkstoffe,
- Ölkomponenten,
- nichtionische Emulgatoren,
- Coemulgatoren,
- Konsistenzgeber,
- Hilfsstoffe wie z. B. Verdicker oder Komplexierungsmittel und/oder
- nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. A-luminiumhydroxyallantoinat, Aluminiumchloridtartrat, Aluminium-Zirkonium-Trichlorohydrat, Aluminium-Zirkonium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
- entzündungshemmende, hautschützende oder wohlriechende ätherische Öle,
- synthetische hautschützende Wirkstoffe und/oder
- öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### Filmbildner

Gebräuchliche Filmbildner sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival® (Climbazole), Ketoconazol®, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon® UD (Protein-Undecylensäurekondensat), Zinkpyrithion, Aluminiumpyrithion und Magnesiumpyrithion / Dipyrithion-Magnesiumsulfat in Frage.

### Quellmittel

Als Quellmittel für wäßrige Phasen können Montmorillonite, Clay Mineralstoffe, Pemulen sowie alkylmodifizierte Carbopoltypen (Goodrich) dienen. Weitere geeignete Polymere bzw. Quellmittel können der Übersicht von R.Lochhead in **Cosm.Toil. 108, 95 (1993)** entnommen werden.

### Spezielle Wirkstoffe

Als Insekten-Repellentien kommen N,N-Diethyl-m-toluamid, 1,2-Pentandiol oder Ethyl Butylacetylaminopropionate in Frage. Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Hydrotrope

Zur Verbesserung des Fließverhaltens können ferner Hydrotrope, wie beispielsweise Ethanol, Isopropylalkohol, oder Polyole eingesetzt werden. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine® bekannten Silberkomplexe und die in Anlage 6, Teil A und B der Kosmetikverordnung aufgeführten weiteren Stoffklassen.

### Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Stermanisöl, Kümmelöl, Eukalyptusöl, Fenchelöl, Citronenöl, Wintergrünöl, Nelkenöl, Menthol und dergleichen in Frage.

### Farbstoffe

Als Farbstoffe können die für kosmetische Zwecke geeigneten und zugelassenen Substanzen verwendet werden, wie sie beispielsweise in der Publikation "**Kosmetische Färbemittel" der Farbstoffkommission der Deutschen Forschungsgemeinschaft, Verlag Chemie, Weinheim, 1984, S.81-106** zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Wasch-, Spül-, Reinigungs- und Avivagemittel

Die erfindungsgemäßen Wasch-, Spül-, Reinigungs- und Avivagemittel können flüssige Zubereitungen darstellen oder auch in fester Form vorliegen. Handelt es sich um Flüssigprodukte, so werden diese in der Regel durch einfaches Vermischen der Komponenten hergestellt. Beide Produkttypen können des weiteren typische Hilfs- und Zusatzstoffe enthalten, wie beispielsweise anionische, nichtionische, kationische, amphotere oder zwitterionische Tenside (wie sie schon oben beschrieben worden sind), Builder, Co-Builder, öl- und fettlösende Stoffe, Bleichmittel, Bleichaktivatoren, Vergrauungsinhibitoren, Enzyme, Enzymstabilisatoren, Optische Aufheller, Polymere, Entschäumer, Sprengmittel, Duftstoffe, anorganische Salze und dergleichen, wie sie im folgenden näher erläutert werden.

### Builder

Die erfindungsgemäßen Wasch-, Spül-, Reinigungs- und Avivagemittel können des weiteren zusätzliche anorganische und organische Buildersubstanzen beispielsweise in Mengen von 10 bis 50 und vorzugsweise 15 bis 35 Gew.-% - bezogen auf die Mittel - enthalten, wobei als anorganische Buildersubstanzen hauptsächlich Zeolithe kristalline Schichtsilicate, amorphe Silicate und - soweit zulässig- auch Phosphate, wie z.B. Tripolyphosphat zum Einsatz kommen. Die Menge an Co-Builder ist dabei auf die bevorzugten Mengen an Phosphaten anzurechnen.

### • Zeolithe

Der als Waschmittelbuilder häufig eingesetzte feinkristalline, synthetische und gebundenes Wasser enthaltende Zeolith ist vorzugsweise Zeolith A und/oder P. Als Zeolith P wird beispielsweise Zeolith MAP^{(R)} (Handelsprodukt der Firma Crosfield) besonders bevorzugt. Geeignet sind jedoch auch Zeolith X sowie Mischungen aus A, X und/oder P wie auch Y. Von besonderem Interesse ist auch ein cokristallisiertes Natrium/Kalium-Aluminiumsilicat aus Zeolith A und Zeolith X, welches als VEGOBOND AX® (Handelsprodukt der Firma Condea Augusta S.p.A.) im Handel erhältlich ist. Der Zeolith kann als sprühgetrocknetes Pulver oder auch als ungetrocknete, von ihrer Herstellung noch feuchte, stabilisierte Suspension zum Einsatz kommen. Für den Fall, dass der Zeolith als Suspension eingesetzt wird, kann diese geringe Zusätze an nichtionischen Tensiden als Stabilisatoren enthalten, beispielsweise 1 bis 3 Gew.-%, bezogen auf Zeolith, an ethoxylierten C₁₂₋C₁₈-Fettalkoholen mit 2 bis 5 Ethylenoxidgruppen, C₁₂-C₁₄-Fettalkoholen mit 4 bis 5 Ethylenoxidgruppen oder ethoxylierten Isotridecanolen. Geeignete Zeolithe weisen eine mittlere Teilchengröße von weniger als 10 µm (Volumenverteilung; Meßmethode: Coulter Counter) auf und enthalten vorzugsweise 18 bis 22 Gew.-%, insbesondere 20 bis 22 Gew.-% an gebundenem Wasser.

### • Schichtsilicate

Geeignete Substitute bzw. Teilsubstitute für Phosphate und Zeolithe sind kristalline, schichtförmige Natriumsilicate der allgemeinen Formel NaMSiₓO₂ₓ₊₁·yH₂O, wobei M Natrium oder Wasserstoff bedeutet, x eine Zahl von 1,9 bis 4 und y eine Zahl von 0 bis 20 ist und bevorzugte Werte für x 2, 3 oder 4 sind. Bevorzugte kristalline Schichtsilicate der angegebenen Formel sind solche, in denen M für Natrium steht und x die Werte 2 oder 3 annimmt. Insbesondere sind sowohl β- als auch δ-Natriumdisilicate Na₂Si₂O₅·yH₂O bevorzugt. Ihre Verwendbarkeit ist nicht auf eine spezielle Zusammensetzung bzw. Strukturformel beschränkt. Bevorzugt sind hier jedoch Smectite, insbesondere Bentonite. Geeignete Schichtsilicate, die zur Gruppe der mit Wasser quellfähigen Smectite zählen, sind z.B. solche der allgemeinen Formeln

| | |
|---|---|
| (OH)₄Si_{8-y}Al_{y}(MgₓAl₄₋ₓ)O₂₀ | Montmorrilonit |
| (OH)₄Si_{8-y}Al_{y}(Mg_{6-z}Li_{z})O₂₀ | Hectorit |
| (OH)₄Si_{8-y}Al_{y}(Mg_{6-z} Al_{z})O₂₀ | Saponit |

mit x = 0 bis 4, y = 0 bis 2, z = 0 bis 6. Zusätzlich kann in das Kristallgitter der Schichtsilicate gemäß den vorstehenden Formeln geringe Mengen an Eisen eingebaut sein. Ferner können die Schichtsilicate aufgrund ihrer ionenaustauschenden Eigenschaften Wasserstoff-, Alkali-, Erdalkaliionen, insbesondere Na⁺ und Ca²⁺ enthalten. Die Hydratwassermenge liegt meist im Bereich von 8 bis 20 Gew.-% und ist vom Quellzustand bzw. von der Art der Bearbeitung abhängig. Vorzugsweise werden Schichtsilicate verwendet, die aufgrund einer Alkalibehandlung weitgehend frei von Calciumionen und stark färbenden Eisenionen sind.

Zu den bevorzugten Buildersubstanzen gehören auch amorphe Natriumsilicate mit einem Modul Na₂O : SiO₂ von 1 : 2 bis 1 : 3,3, vorzugsweise von 1 : 2 bis 1 : 2,8 und insbesondere von 1 : 2 bis 1 : 2,6, welche löseverzögert sind und Sekundärwascheigenschaften aufweisen. Die Löseverzögerung gegenüber herkömmlichen amorphen Natriumsilicaten kann dabei auf verschiedene Weise, beispielsweise durch Oberflächenbehandlung, Compoundierung, Kompaktierung/Verdichtung oder durch Übertrocknung hervorgerufen worden sein. Im Rahmen dieser Erfindung wird unter dem Begriff "amorph" auch "röntgenamorph" verstanden. Dies heißt, dass die Silicate bei Röntgenbeugungsexperimenten keine scharfen Röntgenreflexe liefern, wie sie für kristalline Substanzen typisch sind, sondern allenfalls ein oder mehrere Maxima der gestreuten Röntgenstrahlung, die eine Breite von mehreren Gradeinheiten des Beugungswinkels aufweisen. Es kann jedoch sehr wohl sogar zu besonders guten Buildereigenschaften führen, wenn die Silicatpartikel bei Elektronenbeugungsexperimenten verwaschene oder sogar scharfe Beugungsmaxima liefern. Dies ist so zu interpretieren, dass die Produkte mikrokristalline Bereiche der Größe 10 bis einige Hundert nm aufweisen, wobei Werte bis max. 50 nm und insbesondere bis max. 20 nm bevorzugt sind. Insbesondere bevorzugt sind verdichtete/kompaktierte amorphe Silicate, compoundierte amorphe Silicate und übertrocknete röntgenamorphe Silicate.

### • Phosphate

Selbstverständlich ist auch ein Einsatz der allgemein bekannten Phosphate als Buildersubstanzen möglich, sofern ein derartiger Einsatz nicht aus ökologischen Gründen vermieden werden sollte. Geeignet sind insbesondere die Natriumsalze der Orthophosphate, der Pyrophosphate und insbesondere der Tripolyphosphate. Ihr Gehalt beträgt im allgemeinen nicht mehr als 25 Gew.-%, vorzugsweise nicht mehr als 20 Gew.-%, jeweils bezogen auf das fertige Mittel. In einigen Fällen hat es sich gezeigt, dass insbesondere Tripolyphosphate schon in geringen Mengen bis maximal 10 Gew.-%, bezogen auf das fertige Mittel, in Kombination mit anderen Buildersubstanzen zu einer synergistischen Verbesserung des Sekundärwaschvermögens führen.

### Co-Builder

Brauchbare organische Gerüstsubstanzen, die als Co-Builder in Frage kommen, sind beispielsweise die in Form ihrer Natriumsalze einsetzbaren Polycarbonsäuren, wie Zitronensäure, Adipinsäure, Bernsteinsäure, Glutarsäure, Weinsäure, Zuckersäuren, Aminocarbonsäuren, Nitrilotriessigsäure (NTA), sofern ein derartiger Einsatz aus ökologischen Gründen nicht zu beanstanden ist, sowie Mischungen aus diesen. Bevorzugte Salze sind die Salze der Polycarbonsäuren wie Zitronensäure, Adipinsäure, Bernsteinsäure, Glutarsäure, Weinsäure, Zuckersäuren und Mischungen aus diesen. Auch die Säuren an sich können eingesetzt werden. Die Säuren besitzen neben ihrer Builderwirkung typischerweise auch die Eigenschaft einer Säuerungskomponente und dienen somit auch zur Einstellung eines niedrigeren und milderen pH-Wertes von Wasch- oder Reinigungsmitteln. Insbesondere sind hierbei Zitronensäure, Bemsteinsäure, Glutarsäure, Adipinsäure, Gluconsäure und beliebige Mischungen aus diesen zu nennen.

### • Dextrine

Weitere geeignete organische Buildersubstanzen sind Dextrine, beispielsweise Oligomere bzw. Polymere von Kohlenhydraten, die durch partielle Hydrolyse von Stärken erhalten werden können. Die Hydrolyse kann nach üblichen, beispielsweise säure- oder enzymkatalysierten Verfahren durchgeführt werden. Vorzugsweise handelt es sich um Hydrolyseprodukte mit mittleren Molmassen im Bereich von 400 bis 500 000. Dabei ist ein Polysaccharid mit einem Dextrose-Äquivalent (DE) im Bereich von 0,5 bis 40, insbesondere von 2 bis 30 bevorzugt, wobei DE ein gebräuchliches Maß für die reduzierende Wirkung eines Polysaccharids im Vergleich zu Dextrose, welche ein DE von 100 besitzt, ist. Brauchbar sind sowohl Maltodextrine mit einem DE zwischen 3 und 20 und Trockenglucosesirupe mit einem DE zwischen 20 und 37 als auch sogenannte Gelbdextrine und Weissdextrine mit höheren Molmassen im Bereich von 2 000 bis 30 000. Bei den oxidierten Derivaten derartiger Dextrine handelt es sich um deren Umsetzungsprodukte mit Oxidationsmitteln, welche in der Lage sind, mindestens eine Alkoholfunktion des Saccharidrings zur Carbonsäurefunktion zu oxidieren.

### • Succinate

Weitere geeignete Cobuilder sind Oxydisuccinate und andere Derivate von Disuccinaten, vorzugsweise Ethylendiamindisuccinat. Besonders bevorzugt sind in diesem Zusammenhang auch Glycerindisuccinate und Glycerintrisuccinate. Geeignete Einsatzmengen liegen in zeolithhaltigen und/oder silicathaltigen Formulierungen bei 3 bis 15 Gew.-%. Weitere brauchbare organische Cobuilder sind beispielsweise acetylierte Hydroxycarbonsäuren bzw. deren Salze, welche gegebenenfalls auch in Lactonform vorliegen können und welche mindestens 4 Kohlenstoffatome und mindestens eine Hydroxygruppe sowie maximal zwei Säuregruppen enthalten.

### • Polycarboxylate

Geeignete polymere Polycarboxylate sind beispielsweise die Natriumsalze der Polyacrylsäure oder der Polymethacrylsäure, beispielsweise solche mit einer relativen Molekülmasse von 800 bis 150 000 (auf Säure bezogen und jeweils gemessen gegen Polystyrolsulfonsäure). Geeignete copolymere Polycarboxylate sind insbesondere solche der Acrylsäure mit Methacrylsäure und der Acrylsäure oder Methacrylsäure mit Maleinsäure. Als besonders geeignet haben sich Copolymere der Acrylsäure mit Maleinsäure erwiesen, die 50 bis 90 Gew.-% Acrylsäure und 50 bis 10 Gew.-% Maleinsäure enthalten. Ihre relative Molekülmasse, bezogen auf freie Säuren, beträgt im allgemeinen 5 000 bis 200 000, vorzugsweise 10 000 bis 120 000 und insbesondere 50 000 bis 100 000 (jeweils gemessen gegen Polystyrolsulfonsäure). Die (co-)polymeren Polycarboxylate können entweder als Pulver oder als wässrige Lösung eingesetzt werden, wobei 20 bis 55 Gew.-%ige wässrige Lösungen bevorzugt sind. Granulare Polymere werden zumeist nachträglich zu einem oder mehreren Basisgranulaten zugemischt. Insbesondere bevorzugt sind auch biologisch abbaubare Polymere aus mehr als zwei verschiedenen Monomereinheiten. Ebenso sind als weitere bevorzugte Buildersubstanzen polymere Aminodicarbonsäuren, deren Salze oder deren Vorläufersubstanzen zu nennen. Besonders bevorzugt sind Polyasparaginsäuren bzw. deren Salze und Derivate.

### • Polyacetale

Weitere geeignete Buildersubstanzen sind Polyacetale, welche durch Umsetzung von Dialdehyden mit Polyolcarbonsäuren, welche 5 bis 7 C-Atome und mindestens 3 Hydroxylgruppen aufweisen. Bevorzugte Polyacetale werden aus Dialdehyden wie Glyoxal, Glutaraldehyd, Terephthalaldehyd sowie deren Gemischen und aus Polyolcarbonsäuren wie Gluconsäure und/oder Glucoheptonsäure erhalten.

### Öl- und fettlösende Stoffe

Zusätzlich können die Mittel auch Komponenten enthalten, welche die Öl- und Fett-Auswaschbarkeit aus Textilien positiv beeinflussen. Zu den bevorzugten öl- und fettlösenden Komponenten zählen beispielsweise nichtionische Celluloseether wie Methylcellulose und Methylhydroxypropylcellulose mit einem Anteil an Methoxyl-Gruppen von 15 bis 30 Gew.-% und an Hydroxypropoxyl-Gruppen von 1 bis 15 Gew.-%, jeweils bezogen auf den nichtionischen Celluloseether, sowie die aus dem Stand der Technik bekannten Polymere der Phthalsäure und/oder der Terephthalsäure bzw. von deren Derivaten, insbesondere Polymere aus Ethylenterephthalaten und/oder Polyethylenglykolterephthalaten oder anionisch und/oder nichtionisch modifizierten Derivaten von diesen. Besonders bevorzugt von diesen sind die sulfonierten Derivate der Phthalsäure- und der Terephthalsäure-Polymere.

### Bleichmittel und Bleichaktivatoren

Unter den als Bleichmittel dienenden, in Wasser H₂O₂ liefernden Verbindungen haben das Natriumperborattetrahydrat und das Natriumperboratmonohydrat besondere Bedeutung. Weitere brauchbare Bleichmittel sind beispielsweise Natriumpercarbonat, Peroxypyrophosphate, Citratperhydrate sowie H₂O₂ liefernde persaure Salze oder Persäuren, wie Perbenzoate, Peroxophthalate, Diperazelainsäure, Phthaloiminopersäure oder Diperdodecandisäure. Der Gehalt der Mittel an Bleichmitteln beträgt vorzugsweise 5 bis 35 Gew.-% und insbesondere bis 30 Gew.-%, wobei vorteilhafterweise Perboratmonohydrat oder Percarbonat eingesetzt wird.

Als Bleichaktivatoren können Verbindungen, die unter Perhydrolysebedingungen aliphatische Peroxocarbonsäuren mit vorzugsweise 1 bis 10 C-Atomen, insbesondere 2 bis 4 C-Atomen, und/oder gegebenenfalls substituierte Perbenzoesäure ergeben, eingesetzt werden. Geeignet sind Substanzen, die O- und/oder N-Acylgruppen der genannten C-Atomzahl und/oder gegebenenfalls substituierte Benzoylgruppen tragen. Bevorzugt sind mehrfach acylierte Alkylendiamine, insbesondere Tetraacetylethylendiamin (TAED), acylierte Triazinderivate, insbesondere 1,5-Diacetyl-2,4-dioxohexahydro-1,3,5-triazin (DADHT), acylierte Glykolurile, insbesondere Tetraacetylglykoluril (TAGU), N-Acylimide, insbesondere N-Nonanoylsuccinimid (NOSI), acylierte Phenolsulfonate, insbesondere n-Nonanoyl- oder Isononanoyloxybenzolsulfonat (n- bzw. iso-NOBS), Carbonsäureanhydride, insbesondere Phthalsäureanhydrid, acylierte mehrwertige Alkohole, insbesondere Triacetin, Ethylenglykoldiacetat, 2,5-Diacetoxy-2,5-dihydrofuran, Enolester sowie acetyliertes Sorbitol und Mannitol beziehungsweise deren acylierte Zuckerderivate, insbesondere Pentaacetylglukose (PAG), Pentaacetylfruktose, Tetraacetylxylose und Octaacetyllactose sowie acetyliertes, gegebenenfalls N-alkyliertes Glucamin und Gluconolacton, und/oder N-acylierte Lactame, beispielsweise N-Benzoylcaprolactam. Derartige Bleichaktivatoren sind im üblichen Mengenbereich, vorzugsweise in Mengen von 1 Gew.-% bis 10 Gew.-%, insbesondere 2 Gew.-% bis 8 Gew.-%, bezogen auf gesamtes Mittel, enthalten. Zusätzlich zu den oben aufgeführten konventionellen Bleichaktivatoren oder an deren Stelle können auch Sulfonimine und/oder bleichverstärkende Übergangsmetallsalze beziehungsweise Übergangsmetallkomplexe als sogenannte Bleichkatalysatoren enthalten sein. Zu den in Frage kommenden Übergangsmetallverbindungen gehören insbesondere Mangan-, Eisen-, Kobalt-, Ruthenium- oder Molybdän-Salenkomplexe und deren N-Analogverbindungen, Mangan-, Eisen-, Kobalt-, Ruthenium- oder Molybdän-Carbonylkomplexe, Mangan-, Eisen-, Kobalt-, Ruthenium-, Molybdän-, Titan-, Vanadium- und Kupfer-Komplexe mit stickstoffhaltigen Tripod-Liganden, sowie Kobalt-, Eisen-, Kupfer- und Ruthenium-Aminkomplexe. Bleichverstärkende Übergangsmetallkomplexe, insbesondere mit den Zentralatomen Mn, Fe, Co, Cu, Mo, V, Ti und/oder Ru, werden in üblichen Mengen, vorzugsweise in einer Menge bis zu 1 Gew.-%, insbesondere von 0,0025 Gew.-% bis 0,25 Gew.-% und besonders bevorzugt von 0,01 Gew.-% und besonders bevorzugt von 0,01 Gew.-% bis 0,1 Gew.-%, jeweils bezogen auf gesamtes Mittel, eingesetzt.

### Enzyme und Enzymstabilisatoren

Als Enzyme kommen insbesondere solche aus der Klasse der Hydrolasen, wie der Proteasen, Esterasen, Lipasen bzw. lipolytisch wirkenden Enzyme, Amylasen, Cellulasen bzw. andere Glykosylhydrolasen und Gemische der genannten Enzyme in Frage. Alle diese Hydrolasen tragen in der Wäsche zur Entfernung von Verfleckungen, wie protein-, fett- oder stärkehaltigen Verfleckungen, und Vergrauungen bei. Cellulasen und andere Glykosylhydrolasen können durch das Entfernen von Pilling und Mikrofibrillen zur Farberhaltung und zur Erhöhung der Weichheit des Textils beitragen. Zur Bleiche bzw. zur Hemmung der Farbübertragung können auch Oxidoreduktasen eingesetzt werden. Besonders gut geeignet sind aus Bakterienstämmen oder Pilzen, wie Bacillus subtilis, Bacillus licheniformis, Streptomyces griseus und Humicola insolens gewonnene enzymatische Wirkstoffe. Vorzugsweise werden Proteasen vom Subtilisin-Typ und insbesondere Proteasen, die aus Bacillus lentus gewonnen werden, eingesetzt. Dabei sind Enzymmischungen, beispielsweise aus Protease und Amylase oder Protease und Lipase bzw. lipolytisch wirkenden Enzymen oder Protease und Cellulase oder aus Cellulase und Lipase bzw. lipolytisch wirkenden Enzymen oder aus Protease, Amylase und Lipase bzw. lipolytisch wirkenden Enzymen oder Protease, Lipase bzw. lipolytisch wirkenden Enzymen und Cellulase, insbesondere jedoch Protease- und/oder Lipase-haltige Mischungen bzw. Mischungen mit lipolytisch wirkenden Enzymen von besonderem Interesse. Beispiele für derartige lipolytisch wirkende Enzyme sind die bekannten Cutinasen. Auch Peroxidasen oder O-xidasen haben sich in einigen Fällen als geeignet erwiesen. Zu den geeigneten Amylasen zählen insbesondere α-Amylasen, Iso-Amylasen, Pullulanasen und Pektinasen. Als Cellulasen werden vorzugsweise Cellobiohydrolasen, Endoglucanasen und β-Glucosidasen, die auch Cellobiasen genannt werden, bzw. Mischungen aus diesen eingesetzt. Da sich die verschiedenen Cellulase-Typen durch ihre CMCase- und Avicelase-Aktivitäten unterscheiden, können durch gezielte Mischungen der Cellulasen die gewünschten Aktivitäten eingestellt werden.

Die Enzyme können an Trägerstoffen adsorbiert und/oder in Hüllsubstanzen eingebettet sein, um sie gegen vorzeitige Zersetzung zu schützen. Der Anteil der Enzyme, Enzymmischungen oder Enzymgranulate kann beispielsweise etwa 0,1 bis 5 Gew.-%, vorzugsweise 0,1 bis etwa 2 Gew.-% betragen.

Zusätzlich zu den mono- und polyfunktionellen Alkoholen können die Mittel weitere Enzymstabilisatoren enthalten. Beispielsweise können 0,5 bis 1 Gew.-% Natriumformiat eingesetzt werden. Möglich ist auch der Einsatz von Proteasen, die mit löslichen Calciumsalzen und einem Calciumgehalt von vorzugsweise etwa 1,2 Gew.-%, bezogen auf das Enzym, stabilisiert sind. Außer Calciumsalzen dienen auch Magnesiumsalze als Stabilisatoren. Besonders vorteilhaft ist jedoch der Einsatz von Borverbindungen, beispielsweise von Borsäure, Boroxid, Borax und anderen Alkalimetallboraten wie den Salzen der Orthoborsäure (H₃BO₃), der Metaborsäure (HBO₂) und der Pyroborsäure (Tetraborsäure H₂B₄O₇).

### Vergrauungsinhibitoren

Vergrauungsinhibitoren haben die Aufgabe, den von der Faser abgelösten Schmutz in der Flotte suspendiert zu halten und so das Wiederaufziehen des Schmutzes zu verhindern. Hierzu sind wasserlösliche Kolloide meist organischer Natur geeignet, beispielsweise die wasserlöslichen Salze polymerer Carbonsäuren, Leim, Gelatine, Salze von Ethercarbonsäuren oder E-thersulfonsäuren der Stärke oder der Cellulose oder Salze von sauren Schwefelsäureestern der Cellulose oder der Stärke. Auch wasserlösliche, saure Gruppen enthaltende Polyamide sind für diesen Zweck geeignet. Weiterhin lassen sich lösliche Stärkepräparate und andere als die obengenannten Stärkeprodukte verwenden, z.B. abgebaute Stärke, Aldehydstärken usw.. Auch Polyvinylpyrrolidon ist brauchbar. Bevorzugt werden jedoch Celluloseether, wie Carboxymethylcellulose (Na-Salz), Methylcellulose, Hydroxyalkylcellulose und Mischether, wie Methylhydroxyethylcellulose, Methylhydroxypropylcellulose, Methylcarboxymethylcellulose und deren Gemische, sowie Polyvinylpyrrolidon beispielsweise in Mengen von 0,1 bis 5 Gew.-%, bezogen auf die Mittel, eingesetzt.

### Optische Aufheller

Die Mittel können als optische Aufheller Derivate der Diaminostilbendisulfonsäure bzw. deren Alkalimetallsalze enthalten. Geeignet sind z.B. Salze der 4,4'-Bis(2-anilino-4-morpholino-1,3,5-triazinyl-6-amino)stilben-2,2'-disulfonsäure oder gleichartig aufgebaute Verbindungen, die anstelle der Morpholino-Gruppe eine Diethanolaminogruppe, eine Methylaminogruppe, eine Anilinogruppe oder eine 2-Methoxyethylaminogruppe tragen. Weiterhin können Aufheller vom Typ der substituierten Diphenylstyryle anwesend sein, z.B. die Alkalisalze des 4,4'-Bis(2-sulfostyryl)-diphenyls, 4,4'-Bis(4-chlor-3-sulfostyryl)-diphenyls, oder 4-(4-Chlorstyryl)-4'-(2-sulfostyryl)-diphenyls. Auch Gemische der vorgenannten Aufheller können verwendet werden. Einheitlich weiße Granulate werden erhalten, wenn die Mittel außer den üblichen Aufhellern in üblichen Mengen, beispielsweise zwischen 0,1 und 0,5 Gew.-%, vorzugsweise zwischen 0,1 und 0,3 Gew.-%, auch geringe Mengen, beispielsweise 10⁻⁶ bis 10⁻³ Gew.-%, vorzugsweise um 10⁻⁵ Gew.-%, eines blauen Farbstoffs enthalten. Ein besonders bevorzugter Farbstoff ist Tinolux® (Handelsprodukt der Ciba-Geigy).

### Polymere

Als schmutzabweisende Polymere ("soil repellants") kommen solche Stoffe in Frage, die vorzugsweise Ethylenterephthalat- und/oder Polyethylenglycolterephthalatgruppen enthalten, wobei das Molverhältnis Ethylenterephthalat zu Polyethylenglycolterephthalat im Bereich von 50 : 50 bis 90 : 10 liegen kann. Das Molekulargewicht der verknüpfenden Polyethylenglycoleinheiten liegt insbesondere im Bereich von 750 bis 5000, d.h., der Ethoxylierungsgrad der Polyethylenglycolgruppenhaltigen Polymere kann ca. 15 bis 100 betragen. Die Polymeren zeichnen sich durch ein durchschnittliches Molekulargewicht von etwa 5000 bis 200.000 aus und können eine Block-, vorzugsweise aber eine Random-Struktur aufweisen. Bevorzugte Polymere sind solche mit Molverhältnissen Ethylenterephthalat/Polyethylenglycolterephthalat von etwa 65 : 35 bis etwa 90 : 10, vorzugsweise von etwa 70 : 30 bis 80 : 20. Weiterhin bevorzugt sind solche Polymeren, die verknüpfende Polyethylenglycoleinheiten mit einem Molekulargewicht von 750 bis 5000, vorzugsweise von 1000 bis etwa 3000 und ein Molekulargewicht des Polymeren von etwa 10.000 bis etwa 50.000 aufweisen. Beispiele für handelsübliche Polymere sind die Produkte Milease® T (ICI) oder Repelotex® SRP 3 (Rhöne-Poulenc).

### Entschäumer

Als Entschäumer können wachsartige Verbindungen eingesetzt werden. Als "wachsartig" werden solche Verbindungen verstanden, die einen Schmelzpunkt bei Atmosphärendruck über 25 °C (Raumtemperatur), vorzugsweise über 50 °C und insbesondere über 70 °C aufweisen. Die wachsartigen Entschäumersubstanzen sind in Wasser praktisch nicht löslich, d.h. bei 20 °C weisen sie in 100 g Wasser eine Löslichkeit unter 0,1 Gew.-% auf. Prinzipiell können alle aus dem Stand der Technik bekannten wachsartigen Entschäumersubstanzen enthalten sein. Geeignete wachsartige Verbindungen sind beispielsweise Bisamide, Fettalkohole, Fettsäuren, Carbonsäureester von ein- und mehrwertigen Alkoholen sowie Paraffinwachse oder Mischungen derselben. Alternativ können natürlich auch die für diesen Zweck bekannten Silikonverbindungen eingesetzt werden.

### • Paraffinwachse

Geeignete Paraffinwachse stellen im allgemeinen ein komplexes Stoffgemisch ohne scharfen Schmelzpunkt dar. Zur Charakterisierung bestimmt man üblicherweise seinen Schmelzbereich durch Differential-Thermo-Analyse (DTA) und/oder seinen Erstarrungspunkt. Darunter versteht man die Temperatur, bei der das Paraffin durch langsames Abkühlen aus dem flüssigen in den festen Zustand übergeht. Dabei sind bei Raumtemperatur vollständig flüssige Paraffine, das heißt solche mit einem Erstarrungspunkt unter 25 °C, erfindungsgemäß nicht brauchbar. Zu den Weichwachsen, die einen Schmelzpunkt im Bereich von 35 bis 50 °C aufweisen, zählen vorzugsweise der Gruppe der Petrolate und deren Hydrierprodukte. Sie setzen sich aus mikrokristallinen Paraffinen und bis zu 70 Gew.-% Öl zusammen, besitzen eine salbenartige bis plastisch feste Konsistenz und stellen bitumenfreie Rückstände aus der Erdölverarbeitung dar. Besonders bevorzugt sind Destillationsrückstände (Petrolatumstock) bestimmter paraffinbasischer und gemischtbasischer Rohöle, die zu Vaseline weiterverarbeitet werden. Vorzugsweise handelt es sich weiterhin um aus Destillationsrückständen paraffin- und gemischtbasyischer Rohöle und Zylinderöldestillate mittels Lösungsmittel abgeschiedene bitumenfreie, ölartige bis feste Kohlenwasserstoffe. Sie sind von halbfester, zügiger, klebriger bis plastisch-fester Konsistenz und besitzen Schmelzpunkte zwischen 50 und 70 °C. Diese Petrolate stellen die wichtigste Ausgangsbasis für die Herstellung von Mikrowachsen dar. Weiterhin geeignet sind die aus hochviskosen, paraffinhaltigen Schmieröldestillaten bei der Entparaffinierung abgeschiedenen festen Kohlenwasserstoffen mit Schmelzpunkten zwischen 63 und 79 °C. Bei diesen Petrolaten handelt es sich um Gemische aus mikrokristallinen Wachsen und hochschmelzenden n-Paraffinen. Eingesetzt werden können beispielsweise Paraffinwachsgemische aus beispielsweise 26 Gew.-% bis 49 Gew.-% mikrokristallinem Paraffinwachs mit einem Erstarrungspunkt von 62 °C bis 90 °C, 20 Gew.-% bis 49 Gew.-% Hartparaffin mit einem Erstarrungspunkt von 42 °C bis 56 °C und 2 Gew.-% bis 25 Gew.-% Weichparaffin mit einem Erstarrungspunkt von 35 °C bis 40 °C. Vorzugsweise werden Paraffine bzw. Paraffingemische verwendet, die im Bereich von 30 °C bis 90 °C erstarren. Dabei ist zu beachten, dass auch bei Raumtemperatur fest erscheinende Paraffinwachsgemische unterschiedliche Anteile an flüssigem Paraffin enthalten können. Bei den erfindungsgemäß brauchbaren Paraffinwachsen liegt dieser Flüssiganteil so niedrig wie möglich und fehlt vorzugsweise ganz. So weisen besonders bevorzugte Paraffinwachsgemische bei 30 °C einen Flüssiganteil von unter 10 Gew.-%, insbesondere von 2 Gew.-% bis 5 Gew.-%, bei 40 °C einen Flüssiganteil von unter 30 Gew.-%, vorzugsweise von 5 Gew.-% bis 25 Gew.-% und insbesondere von 5 Gew.-% bis 15 Gew.-%, bei 60 °C einen Flüssiganteil von 30 Gew.-% bis 60 Gew.-%, insbesondere von 40 Gew.-% bis 55 Gew.-%, bei 80 °C einen Flüssiganteil von 80 Gew.-% bis 100 Gew.-%, und bei 90 °C einen Flüssiganteil von 100 Gew.-% auf. Die Temperatur, bei der ein Flüssiganteil von 100 Gew.-% des Paraffinwachses erreicht wird, liegt bei besonders bevorzugten Paraffinwachsgemischen noch unter 85 °C, insbesondere bei 75 °C bis 82 °C. Bei den Paraffinwachsen kann es sich um Petrolatum, mikrokristalline Wachse bzw. hydrierte oder partiell hydrierte Paraffinwachse handeln.

### • Bisamide

Geeignete Bisamide als Entschäumer sind solche, die sich von gesättigten Fettsäuren mit 12 bis 22, vorzugsweise 14 bis 18 C-Atomen sowie von Alkylendiaminen mit 2 bis 7 C-Atomen ableiten. Geeignete Fettsäuren sind Laurin-, Myristin-, Stearin-, Arachin- und Behensäure sowie deren Gemische, wie sie aus natürlichen Fetten beziehungsweise gehärteten Ölen, wie Talg oder hydriertem Palmöl, erhältlich sind. Geeignete Diamine sind beispielsweise Ethylendiamin, 1,3-Propylendiamin, Tetramethylendiamin, Pentamethylendiamin, Hexamethylendiamin, p-Phenylendiamin und Toluylendiamin. Bevorzugte Diamine sind Ethylendiamin und Hexamethylendiamin. Besonders bevorzugte Bisamide sind Bismyristoylethylendiamin, Bispalmitoylethylendiamin, Bisstearoylethylendiamin und deren Gemische sowie die entsprechenden Derivate des Hexamethylendiamins.

### • Carbonsäureester

Geeignete Carbonsäureester als Entschäumer leiten sich von Carbonsäuren mit 12 bis 28 Kohlenstoffatomen ab. Insbesondere handelt es sich um Ester von Behensäure, Stearinsäure, Hydroxystearinsäure, Ölsäure, Palmitinsäure, Myristinsäure und/oder Laurinsäure. Der Alkoholteil des Carbonsäureesters enthält einen ein- oder mehrwertigen Alkohol mit 1 bis 28 Kohlenstoffatomen in der Kohlenwasserstoffkette. Beispiele von geeigneten Alkoholen sind Behenylalkohol, Arachidylalkohol, Kokosalkohol, 12-Hydroxystearylalkohol, Oleylalkohol und Laurylalkohol sowie Ethylenglykol, Glycerin, Polyvinylalkohol, Saccharose, Erythrit, Pentaerythrit, Sorbitan und/oder Sorbit. Bevorzugte Ester sind solche von Ethylenglykol, Glycerin und Sorbitan, wobei der Säureteil des Esters insbesondere aus Behensäure, Stearinsäure, Ölsäure, Palmitinsäure oder Myristinsäure ausgewählt wird. In Frage kommende Ester mehrwertiger Alkohole sind beispielsweise Xylitmonopalmitat, Pentarythritmonostearat, Glycerin-monostearat, Ethylenglykolmonostearat und Sorbitanmonostearat, Sorbitanpalmitat, Sorbitanmonolaurat, Sorbitandilaurat, Sorbitandistearat, Sorbitandibehenat, Sorbitandioleat sowie gemischte Talgalkylsorbitanmono- und -diester. Brauchbare Glycerinester sind die Mono-, Di- oder Triester von Glycerin und genannten Carbonsäuren, wobei die Mono- oder Dieester bevorzugt sind. Glycerinmonostearat, Glycerinmonooleat, Glycerinmonopalmitat, Glycerinmonobehenat und Glycerindistearat sind Beispiele hierfür. Beispiele für geeignete natürliche Ester als Entschäumer sind Bienenwachs, das hauptsächlich aus den Estern CH₃(CH₂)₂₄COO(CH₂)₂₇CH₃ und CH₃(CH₂)₂₆COO(CH₂)₂₅CH₃ besteht, und Carnaubawachs, das ein Gemisch von Carnaubasäurealkylestem, oft in Kombination mit geringen Anteilen freier Carnaubasäure, weiteren langkettigen Säuren, hochmolekularen Alkoholen und Kohlenwasserstoffen, ist.

### • Carbonsäuren

Geeignete Carbonsäuren als weitere Entschäumerverbindung sind insbesondere Behensäure, Stearinsäure, Ölsäure, Palmitinsäure, Myristinsäure und Laurinsäure sowie deren Gemische, wie sie aus natürlichen Fetten bzw. gegebenenfalls gehärteten Ölen, wie Talg oder hydriertem Palmöl, erhältlich sind. Bevorzugt sind gesättigte Fettsäuren mit 12 bis 22, insbesondere 18 bis 22 C-Atomen. In gleicher Weise können die entsprechenden Fettalkohole gleicher C-Kettenlänge eingesetzt werden.

### • Dialkylether und -ketone

Weiterhin können zusätzlich Dialkylether als Entschäumer enthalten sein. Die Ether können asymmetrisch oder aber symmetrisch aufgebaut sein, d.h. zwei gleiche oder verschiedene Alkylketten, vorzugsweise mit 8 bis 18 Kohlenstoffatomen enthalten. Typische Beispiele sind Di-n-octylether, Di-i-octylether und Di-n-stearylether, insbesondere geeignet sind Dialkylether, die einen Schmelzpunkt über 25 °C, insbesondere über 40 °C aufweisen.Weitere geeignete Entschäumerverbindungen sind Fettketone, die nach den einschlägigen Methoden der präparativen organischen Chemie erhalten werden können. Zu ihrer Herstellung geht man beispielsweise von Carbonsäuremagnesiumsalzen aus, die bei Temperaturen oberhalb von 300 °C unter Abspaltung von Kohlendioxid und Wasser pyrolysiert werden. Geeignete Fettketone sind solche, die durch Pyrolyse der Magnesiumsalze von Laurinsäure, Myristinsäure, Palmitinsäure, Palmitoleinsäure, Stearinsäure, Ölsäure, Elaidinsäure, Petroselinsäure, Arachinsäure, Gadoleinsäure, Behensäure oder Erucasäure hergestellt werden.

### • Fettsäurepolyethylenglycolester

Weitere geeignete Entschäumer sind Fettsäurepolyethylenglykolester, die vorzugsweise durch basisch homogen katalysierte Anlagerung von Ethylenoxid an Fettsäuren erhalten werden. Insbesondere erfolgt die Anlagerung von Ethylenoxid an die Fettsäuren in Gegenwart von Alkanolaminen als Katalysatoren. Der Einsatz von Alkanolaminen, speziell Triethanolamin, führt zu einer äußerst selektiven Ethoxylierung der Fettsäuren, insbesondere dann, wenn es darum geht, niedrig ethoxylierte Verbindungen herzustellen. Innerhalb der Gruppe der Fettsäurepolyethylenglykolester werden solche bevorzugt, die einen Schmelzpunkt über 25 °C, insbesondere über 40 °C aufweisen .

### • Silicone

Geeignete Silicone sind übliche Organopolysiloxane, die einen Gehalt an feinteiliger Kieselsäure, die wiederum auch silaniert sein kann, aufweisen können. Besonders bevorzugt sind Polydiorganosiloxane und insbesondere Polydimethylsiloxane, die aus dem Stand der Technik bekannt sind. Geeignete Polydiorganosiloxane weisen eine nahezu lineare Kette auf und weisen einen Oligomerisierungsgrad von 40 bis 1500 auf. Beispiele für geeignete Substituenten sind Methyl, Ethyl, Propyl, Isobutyl, tert. Butyl und Phenyl. Weiterhin geeignet sind amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt. In der Regel enthalten die Silicone im allgemeinen und die Polydiorganosiloxane im besonderen feinteilige Kieselsäure, die auch silaniert sein kann. Insbesondere geeignet sind im Sinne der vorliegenden Erfindung kieselsäurehaltige Dimethylpolysiloxane. Vorteilhafterweise haben die Polydiorganosiloxane eine Viskosität nach Brookfield bei 25 °C (Spindel 1, 10 Upm) im Bereich von 5000 mPas bis 30 000 mPas, insbesondere von 15 000 bis 25 000 mPas. Vorzugsweise werden die Silicone in Form ihrer wässrigen Emulsionen eingesetzt. In der Regel gibt man das Silicon zu vorgelegtem Wasser unter Rühren. Falls gewünscht kann man zur Erhöhung der Viskosität der wässrigen Siliconemulsionen Verdickungsmittel, wie sie aus dem Stand der Technik bekannt sind, zugeben. Diese können anorganischer und/oder organischer Natur sein, besonders bevorzugt werden nichtionische Celluloseether wie Methylcellulose, Ethylcellulose und Mischether wie Methylhydoxyethylcellulose, Methylhydroxypropylcellulose, Methylhydroxybutylcellulose sowie anionische Carboxycellulose-Typen wie das Carboxymethylcellulose-Natriumsalz (Abkürzung CMC). Insbsonders geeignete Verdicker sind Mischungen von CMC zu nicht-ionischen Celluloseethern im Gewichtsverhältnis 80 : 20 bis 40 : 60, insbesondere 75 : 25 bis 60 : 40. In der Regel und besonders bei Zugabe der beschriebenen Verdickermischungen empfehlen sich Einsatzkonzentrationen von cirka 0,5 bis 10, insbesondere von 2,0 bis 6 Gew.-% - berechnet als Verdickermischung und bezogen auf wässrige Siliconemulsion. Die Gehalt an Siliconen der beschriebenen Art in den wässrigen Emulsionen liegt vorteilhafterweise im Bereich von 5 bis 50 Gew.-%, insbesondere von 20 bis 40 Gew.-% - berechnet als Silicone und bezogen auf wässrige Siliconemulsion. Nach einer weiteren vorteilhaften Ausgestaltung erhalten die wässrigen Siliconlösungen als Verdicker Stärke, die aus natürlichen Quellen zugänglich ist, beispielsweise aus Reis, Kartoffeln, Mais und Weizen. Die Stärke ist vorteilhafterweise in Mengen von 0,1 bis zu 50 Gew.-% - bezogen auf Silicon-Emulsion - enthalten und insbesondere in Mischung mit den schon beschriebenen Verdickermischungen aus Natrium-Carboxymethylcellulose und einem nichtionischen Celluloseether in den schon genannten Mengen. Zur Herstellung der wässrigen Siliconemulsionen geht man zweckmäßigerweise so vor, dass man die gegebenenfalls vorhandenen Verdickungsmittel in Wasser vorquellen lässt, bevor die Zugabe der Silicone erfolgt. Das Einarbeiten der Silicone erfolgt zweckmäßigerweise mit Hilfe wirksamer Rühr- und Mischungsvorrichtungen.

Innerhalb der Gruppe der wachsartigen Entschäumer werden besonders bevorzugt die beschriebenen Paraffinwachse alleine als wachsartige Entschäumer eingesetzt oder in Mischung mit einem der anderen wachsartigen Entschäumer, wobei der Anteil der Paraffinwachse in der Mischung vorzugsweise über 50 Gew.-% - bezogen auf wachsartige Entschäumermischung - ausmacht. Die Paraffinwachse können bei Bedarf auf Träger aufgebracht sein. Als Trägermaterial sind alle bekannten anorganischen und/oder organischen Trägermaterialien geeignet. Beispiele für typische anorganische Trägermaterialien sind Alkalicarbonate, Alumosilicate, wasserlösliche Schichtsilicate, Alkalisilicate, Alkalisulfate, beispielsweise Natriumsulfat, und Alkaliphosphate. Bei den Alkalisilicaten handelt es sich vorzugsweise um eine Verbindung mit einem Molverhältnis Alkalioxid zu SiO₂ von 1 : 1,5 bis 1 : 3,5. Die Verwendung derartiger Silicate resultiert in besonders guten Korneigenschaften, insbesondere hoher Abriebsstabilität und dennoch hoher Auflösungsgeschwindigkeit in Wasser. Zu den als Trägermaterial bezeichneten Alumosilicaten gehören insbesondere die Zeolithe, beispielsweise Zeolith NaA und NaX. Zu den als wasserlöslichen Schichtsilicaten bezeichneten Verbindungen gehören beispielsweise amorphes oder kristallines Wasserglas. Weiterhin können Silicate Verwendung finden, welche unter der Bezeichnung Aerosil® oder Sipernat® im Handel sind. Als organische Trägermaterialien kommen zum Beispiel filmbildende Polymere, beispielsweise Polyvinylalkohole, Polyvinylpyrrolidone, Poly(meth)acrylate, Polycarboxylate, Cellulosederivate und Stärke in Frage. Brauchbare Celluloseether sind insbesondere Alkalicarboxymethylcellulose, Methylcellulose, Ethylcellulose, Hydroxyethylcellulose und sogenannte Cellulosemischether, wie zum Beispiel Methylhydroxyethylcellulose und Methyl-Cellulosemischether, wie zum Beispiel Methylhydroxyethylcellulose und Methylhydroxypropylcellulose, sowie deren Mischungen. Besonders geeignete Mischungen sind aus Natrium-Carboxymethylcellulose und Methylcellulose zusammengesetzt, wobei die Carboxymethylcellulose üblicherweise einen Substitutionsgrad von 0,5 bis 0,8 Carboxymethylgruppen pro Anhydroglukoseeinheit und die Methylcellulose einen Substitutionsgrad von 1,2 bis 2 Methylgruppen pro Anhydroglukoseeinheit aufweist. Die Gemische enthalten vorzugsweise Alkalicarboxymethylcellulose und nichtionischen Celluloseether in Gewichtsverhältnissen von 80 : 20 bis 40 : 60, insbesondere von 75 : 25 bis 50 : 50. Als Träger ist auch native Stärke geeignet, die aus Amylose und Amylopectin aufgebaut ist. Als native Stärke wird Stärke bezeichnet, wie sie als Extrakt aus natürlichen Quellen zugänglich ist, beispielsweise aus Reis, Kartoffeln, Mais und Weizen. Native Stärke ist ein handelsübliches Produkt und damit leicht zugänglich. Als Trägermaterialien können einzeln oder mehrere der vorstehend genannten Verbindungen eingesetzt werden, insbesondere ausgewählt aus der Gruppe der Alkalicarbonate, Alkalisulfate, Alkaliphosphate, Zeolithe, wasserlösliche Schichtsilicate, Alkalisilicate, Polycarboxylate, Celluloseether, Polyacrylat/Polymethacrylat und Stärke. Besonders geeignet sind Mischungen von Alkalicarbonaten, insbesondere Natriumcarbonat, Alkalisilicaten, insbesondere Natriumsilicat, Alkalisulfaten, insbesondere Natriumsulfat und Zeolithen.

### Sprengmittel

Die festen Zubereitungen können des weiteren Spreng- oder Desintegrationsmittel enthalten. Hierunter sind Stoffe zu verstehen, die den Formkörpern zugegeben werden, um deren Zerfall beim Inkontaktbringen mit Wasser zu beschleunigen. Diese Stoffe vergrößern bei Wasserzutritt ihr Volumen, wobei einerseits das Eigenvolumen vergrößert (Quellung), andererseits auch über die Freisetzung von Gasen ein Druck erzeugt werden kann, der die Tablette in kleinere Partikel zerfallen lässt. Altbekannte Desintegrationshilfsmittel sind beispielsweise Carbonat/Citronensäure-Systeme, wobei auch andere organische Säuren eingesetzt werden können. Quellende Desintegrationshilfsmittel sind beispielsweise synthetische Polymere wie gegebenenfalls quervernetztes Polyvinylpyrrolidon (PVP) oder natürliche Polymere bzw. modifizierte Naturstoffe wie Cellulose und Stärke und ihre Derivate, Alginate oder Casein-Derivate. Als bevorzugte Desintegrationsmittel werden im Rahmen der vorliegenden Erfindung Desintegrationsmittel auf Cellulosebasis eingesetzt. Reine Cellulose weist die formale Bruttozusammensetzung (C₆H₁₀O₅)ₙ auf und stellt formal betrachtet ein β-1,4-Polyacetal von Cellobiose dar, die ihrerseits aus zwei Molekülen Glucose aufgebaut ist. Geeignete Cellulosen bestehen dabei aus ca. 500 bis 5000 Glucose-Einheiten und haben demzufolge durchschnittliche Molmassen von 50.000 bis 500.000. Als Desintegrationsmittel auf Cellulosebasis verwendbar sind im Rahmen der vorliegenden Erfindung auch Cellulose-Derivate, die durch polymeranaloge Reaktionen aus Cellulose erhältlich sind. Solche chemisch modifizierten Cellulosen umfassen dabei beispielsweise Produkte aus Veresterungen bzw. Veretherungen, in denen Hydroxy-Wasserstoffatome substituiert wurden. Aber auch Cellulosen, in denen die Hydroxylgruppen gegen funktionelle Gruppen, die nicht über ein Sauerstoffatom gebunden sind, ersetzt wurden, lassen sich als Cellulosederivate einsetzen. In die Gruppe der Cellulosederivate fallen beispielsweise Alkalicellulosen, Carboxymethylcellulose (CMC), Celluloseester und -ether sowie Aminocellulosen. Die genannten Cellulosederivate werden vorzugsweise nicht allein als Sprengmittel auf Cellulosebasis eingesetzt, sondern in Mischung mit Cellulose verwendet. Der Gehalt dieser Mischungen an Cellulosederivaten beträgt vorzugsweise unterhalb 50 Gew.-%, besonders bevorzugt unterhalb 20 Gew.-%, bezogen auf das Desintegrationsmittel auf Cellulosebasis. Besonders bevorzugt wird als Desintegrationsmittel auf Cellulosebasis reine Cellulose eingesetzt, die frei von Cellulosederivaten ist. Als weiteres Desintegrationsmittel auf Cellulosebasis oder als Bestandteil dieser Komponente kann mikrokristalline Cellulose verwendet werden. Diese mikrokristalline Cellulose wird durch partielle Hydrolyse von Cellulosen unter solchen Bedingungen erhalten, die nur die amorphen Bereiche (ca. 30% der Gesamt-Cellulosemasse) der Cellulosen angreifen und vollständig auflösen, die kristallinen Bereiche (ca. 70%) aber unbeschadet lassen. Eine nachfolgende Desaggregation der durch die Hydrolyse entstehenden mikrofeinen Cellulosen liefert die mikrokristallinen Cellulosen, die Primärteilchengrößen von ca. 5 µm aufweisen und beispielsweise zu Granulaten mit einer mittleren Teilchengröße von 200 µm kompaktierbar sind. Die Sprengmittel können im Formkörper makroskopisch betrachtet homogen verteilt vorliegen, mikroskopisch gesehen bilden sie jedoch herstellungsbedingt Zonen erhöhter Konzentration. Sprengmittel, die im Sinne der Erfindung zugegen sein können, sind z.B. Kollidon, Alginsäure und deren Alkalisalze, amorphe oder auch teilweise kristalline Schichtsilicate (Bentonite), Polyacrylate, Polyethylenglycole. Die Zubereitungen können die Sprengmittel in Mengen von 0,1 bis 25, vorzugsweise 1 bis 20 und insbesondere 5 bis 15 Gew.-% - bezogen auf die Formkörper enthalten.

### Duftstoffe

Als Parfümöle bzw. Duftstoffe können einzelne Riechstoffverbindungen, z.B. die synthetischen Produkte vom Typ der Ester, Ether, Aldehyde, Ketone, Alkohole und Kohlenwasserstoffe verwendet werden. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8-18 C-Atomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Geraniol, Linalool, Phenylethylalkohol und Terpineol, zu den Kohlenwasserstoffen gehören hauptsächlich die Terpene wie Limonen und Pinen. Bevorzugt werden jedoch Mischungen verschiedener Riechstoffe verwendet, die gemeinsam eine ansprechende Duftnote erzeugen. Solche Parfümöle können auch natürliche Riechstoffgemische enthalten, wie sie aus pflanzlichen Quellen zugänglich sind, z.B. Pine-, Citrus-, Jasmin-, Patchouly-, Rosen- oder Ylang-Ylang-Öl. Ebenfalls geeignet sind Muskateller, Salbeiöl, Kamillenöl, Nelkenöl, Melissenöl, Minzöl, Zimtblätteröl, Lindenblütenöl, Wacholderbeeröl, Vetiveröl, Olibanumöl, Galbanumöl und Labdanumöl sowie Orangenblütenöl, Neroliol, Orangenschalenöl und Sandelholzöl. Die Duftstoffe können direkt in die erfindungsgemäßen Mittel eingearbeitet werden, es kann aber auch vorteilhaft sein, die Duftstoffe auf Träger aufzubringen, welche die Haftung des Parfüms auf der Wäsche verstärken und durch eine langsamere Duftfreisetzung für langanhaltenden Duft der Textilien sorgen. Als solche Trägermaterialien haben sich beispielsweise Cyclodextrine bewährt, wobei die Cyclodextrin-Parfüm-Komplexe zusätzlich noch mit weiteren Hilfsstoffen beschichtet werden können.

### Anorganische Salze

Weitere geeignete Inhaltsstoffe der Mittel sind wasserlösliche anorganische Salze wie Bicarbonate, Carbonate, amorphe Silicate, normale Wassergläser, welche keine herausragenden Buildereigenschaften aufweisen, oder Mischungen aus diesen; insbesondere werden Alkalicarbonat und/oder amorphes Alkalisilicat, vor allem Natriumsilicat mit einem molaren Verhältnis Na₂O : SiO₂ von 1 : 1 bis 1 : 4,5, vorzugsweise von 1 : 2 bis 1 : 3,5, eingesetzt. Der Gehalt an Natriumcarbonat in den Endzubereitungen beträgt dabei vorzugsweise bis zu 40 Gew.-%, vorteilhafterweise zwischen 2 und 35 Gew.-%. Der Gehalt der Mittel an Natriumsilicat (ohne besondere Buildereigenschaften) beträgt im allgemeinen bis zu 10 Gew.-% und vorzugsweise zwischen 1 und 8 Gew.-%. Als Füll- bzw. Stellmittel kann ferner beispielsweise Natriumsulfat in Mengen von 0 bis 10, insbesondere 1 bis 5 Gew.-% - bezogen auf Mittel - enthalten sein

### Herstellung der Waschmittel

Die unter Einsatz der erfindungsgemäßen Zusatzstoffe erhältlichen Waschmittel können in Form von Pulvern, Extrudaten, Granulaten oder Agglomeraten hergestellt bzw. eingesetzt werden. Es kann sich dabei sowohl um Universal- als auch Fein- bzw. Colorwaschmittel, gegebenenfalls in Form von Kompaktaten oder Superkompaktaten handeln. Zur Herstellung solcher Mittel sind die entsprechenden, aus dem Stand der Technik bekannten Verfahren, geeignet. Bevorzugt werden die Mittel dadurch hergestellt, dass verschiedene teilchenförmige Komponenten, die Waschmittelinhaltsstoffe enthalten, miteinander vermischt werden. Die teilchenförmigen Komponenten können durch Sprühtrocknung, einfaches Mischen oder komplexe Granulationsverfahren, beispielsweise Wirbelschichtgranulation, hergestellt werden. Bevorzugt ist dabei insbesondere, dass mindestens eine tensidhaltige Komponente durch Wirbelschichtgranulation hergestellt wird. Weiter kann es insbesondere bevorzugt sein, wenn wässrige Zubereitungen des Alkalisilicats und des Alkalicarbonats gemeinsam mit anderen Waschmittelinhaltsstoffen in einer Trockeneinrichtung versprüht werden, wobei gleichzeitig mit der Trocknung eine Granulation stattfinden kann.

### • Sprühtrocknung

Bei der Trockeneinrichtung, in die die wässrige Zubereitung versprüht wird, kann es sich um beliebige Trockenapparaturen handeln. In einer bevorzugten Verfahrensführung wird die Trocknung als Sprühtrocknung in einem Trockenturm durchgeführt. Dabei werden die wässrigen Zubereitungen in bekannter Weise einem Trocknungsgasstrom in feinverteilter Form ausgesetzt. In Patentveröffentlichungen der Firma Henkel wird eine Ausführungsform der Sprühtrocknung mit überhitztem Wasserdampf beschrieben. Das dort offenbarte Arbeitsprinzip wird hiermit ausdrücklich auch zum Gegenstand der vorliegenden Erfindungsoffenbarung gemacht.

### • Wirbelchichtgranulierung

Eine besonders bevorzugte Möglichkeit zur Herstellung der Mittel besteht darin, die Vorprodukte einer Wirbelschichtgranulierung ("SKET"-Granulierung) zu unterwerfen. Hierunter ist eine Granulierung unter gleichzeitiger Trocknung zu verstehen, die vorzugsweise batchweise oder kontinuierlich erfolgt. Dabei können die Vorprodukte sowohl in getrocknetem Zustand als auch als wässrige Zubereitung eingesetzt werden. Bevorzugt eingesetzte Wirbelschicht-Apparate besitzen Bodenplatten mit Abmessungen von 0,4 bis 5 m. Vorzugsweise wird die Granulierung bei Wirbelluftgeschwindigkeiten im Bereich von 1 bis 8 m/s durchgeführt. Der Austrag der Granulate aus der Wirbelschicht erfolgt vorzugsweise über eine Größenklassierung der Granulate. Die Klassierung kann beispielsweise mittels einer Siebvorrichtung oder durch einen entgegengeführten Luftstrom (Sichterluft) erfolgen, der so reguliert wird, dass erst Teilchen ab einer bestimmten Teilchengröße aus der Wirbelschicht entfernt und kleinere Teilchen in der Wirbelschicht zurückgehalten werden. Üblicherweise setzt sich die einströmende Luft aus der beheizten oder unbeheizten Sichterluft und der beheizten Bodenluft zusammen. Die Bodenlufttemperatur liegt dabei zwischen 80 und 400, vorzugsweise 90 und 350 °C. Vorteilhafterweise wird zu Beginn der Granulierung eine Startmasse, beispielsweise ein Granulat aus einem früheren Versuchsansatz, vorgelegt.

### • Pressagglomeration

In einer anderen, insbesondere wenn Mittel hoher Schüttdichte erhalten werden sollen, bevorzugten Variante werden die Gemische anschließend einem Kompaktierungsschritt unterworfen, wobei weitere Inhaltsstoffe den Mitteln erst nach dem Kompaktierungsschritt zugemischt werden. Die Kompaktierung der Inhaltsstoffe findet in einer bevorzugten Ausführungsform der Erfindung in einem Pressagglomerationsverfahren statt. Der Pressagglomerationsvorgang, dem das feste Vorgemisch (getrocknetes Basiswaschmittel) unterworfen wird, kann dabei in verschiedenen Apparaten realisiert werden. Je nach dem Typ des verwendeten Agglomerators werden unterschiedliche Pressagglomerationsverfahren unterschieden. Die vier häufigsten und im Rahmen der vorliegenden Erfindung bevorzugten Pressagglomerationsverfahren sind dabei die Extrusion, das Walzenpressen bzw. Walzenkompaktieren, das Lochpressen (Pelletieren) und das Tablettieren, so dass im Rahmen der vorliegenden Erfindung bevorzugte Pressagglomerationsvorgänge Extrusions-, Walzenkompaktierungs-, Pelletierungs- oder Tablettierungsvorgänge sind.

Allen Verfahren ist gemeinsam, dass das Vorgemisch unter Druck verdichtet und plastifiziert wird und die einzelnen Partikel unter Verringerung der Porosität aneinandergedrückt werden und aneinander haften. Bei allen Verfahren (bei der Tablettierung mit Einschränkungen) lassen sich die Werkzeuge dabei auf höhere Temperaturen aufheizen oder zur Abführung der durch Scherkräfte entstehenden Wärme kühlen. In allen Verfahren kann als Hilfsmittel zur Verdichtung ein oder mehrere Bindemittel eingesetzt werden. Dabei soll jedoch klargestellt sein, dass an sich immer auch der Einsatz von mehreren, verschiedenen Bindemitteln und Mischungen aus verschiedenen Bindemitteln möglich ist. In einer bevorzugten Ausführungsform der Erfindung wird ein Bindemittel eingesetzt, dass bei Temperaturen bis maximal 130 °C, vorzugsweise bis maximal 100 °C und insbesondere bis 90 °C bereits vollständig als Schmelze vorliegt. Das Bindemittel muss also je nach Verfahren und Verfahrensbedingungen ausgewählt werden oder die Verfahrensbedingungen, insbesondere die Verfahrenstemperatur, müssen - falls ein bestimmtes Bindemittel gewünscht wird - an das Bindemittel angepasst werden.

Der eigentliche Verdichtungsprozess erfolgt dabei vorzugsweise bei Verarbeitungstemperaturen, die zumindest im Verdichtungsschritt mindestens der Temperatur des Erweichungspunkts, wenn nicht sogar der Temperatur des Schmelzpunkts des Bindemittels entsprechen. In einer bevorzugten Ausführungsform der Erfindung liegt die Verfahrenstemperatur signifikant über dem Schmelzpunkt bzw. oberhalb der Temperatur, bei der das Bindemittel als Schmelze vorliegt. Insbesondere ist es aber bevorzugt, dass die Verfahrenstemperatur im Verdichtungsschritt nicht mehr als 20 °C über der Schmelztemperatur bzw. der oberen Grenze des Schmelzbereichs des Bindemittels liegt. Zwar ist es technisch durchaus möglich, auch noch höhere Temperaturen einzustellen; es hat sich aber gezeigt, dass eine Temperaturdifferenz zur Schmelztemperatur bzw. zur Erweichungstemperatur des Bindemittels von 20 °C im allgemeinen durchaus ausreichend ist und noch höhere Temperaturen keine zusätzlichen Vorteile bewirken. Deshalb ist es - insbesondere auch aus energetischen Gründen - besonders bevorzugt, zwar oberhalb, jedoch so nah wie möglich am Schmelzpunkt bzw. an der oberen Temperaturgrenze des Schmelzbereichs des Bindemittels zu arbeiten. Eine derartige Temperaturführung besitzt den weiteren Vorteil, dass auch thermisch empfindliche Rohstoffe, beispielsweise Peroxybleichmittel wie Perborat und/oder Percarbonat, aber auch Enzyme, zunehmend ohne gravierende Aktivsubstanzverluste verarbeitet werden können. Die Möglichkeit der genauen Temperatursteuerung des Binders insbesondere im entscheidenden Schritt der Verdichtung, also zwischen der Vermischung/Homogenisierung des Vorgemisches und der Formgebung, erlaubt eine energetisch sehr günstige und für die temperaturempfindlichen Bestandteile des Vorgemisches extrem schonende Verfahrensführung, da das Vorgemisch nur für kurze Zeit den höheren Temperaturen ausgesetzt ist. In bevorzugten Pressagglomerationsverfahren weisen die Arbeitswerkzeuge des Pressagglomerators (die Schnecke(n) des Extruders, die Walze(n) des Walzenkompaktors sowie die Presswalze(n) der Pelletpresse) eine Temperatur von maximal 150 °C, vorzugsweise maximal 100 °C und insbesondere maximal 75 °C auf und die Verfahrenstemperatur liegt bei 30 °C und insbesondere maximal 20 °C oberhalb der Schmelztemperatur bzw. der oberen Temperaturgrenze des Schmelzbereichs des Bindemittels. Vorzugsweise beträgt die Dauer der Temperatureinwirkung im Kompressionsbereich der Pressagglomeratoren maximal 2 Minuten und liegt insbesondere in einem Bereich zwischen 30 Sekunden und 1 Minute.

Bevorzugte Bindemittel, die allein oder in Mischung mit anderen Bindemitteln eingesetzt werden können, sind Polyethylenglykole, 1,2-Polypropylenglykole sowie modifizierte Polyethylenglykole und Polypropylenglykole. Zu den modifizierten Polyalkylenglykolen zählen insbesondere die Sulfate und/oder die Disulfate von Polyethylenglykolen oder Polypropylenglykolen mit einer relativen Molekülmasse zwischen 600 und 12 000 und insbesondere zwischen 1 000 und 4 000. Eine weitere Gruppe besteht aus Mono- und/oder Disuccinaten der Polyalkylenglykole, welche wiederum relative Molekülmassen zwischen 600 und 6 000, vorzugsweise zwischen 1 000 und 4 000 aufweisen. Im Rahmen dieser Erfindung zählen zu Polyethylenglykolen solche Polymere, bei deren Herstellung neben Ethylenglykol ebenso C₃-C₅₋Glykole sowie Glycerin und Mischungen aus diesen als Startmoleküle eingesetzt werden. Ferner werden auch ethoxylierte Derivate wie Trimethylolpropan mit 5 bis 30 EO umfaßt. Die vorzugsweise eingesetzten Polyethylenglykole können eine lineare oder verzweigte Struktur aufweisen, wobei insbesondere lineare Polyethylenglykole bevorzugt sind. Zu den insbesondere bevorzugten Polyethylenglykolen gehören solche mit relativen Molekülmassen zwischen 2 000 und 12 000, vorteilhafterweise um 4 000, wobei Polyethylenglykole mit relativen Molekülmassen unterhalb 3 500 und oberhalb 5 000 insbesondere in Kombination mit Polyethylenglykolen mit einer relativen Molekülmasse um 4 000 eingesetzt werden können und derartige Kombinationen vorteilhafterweise zu mehr als 50 Gew.-%, bezogen auf die gesamte Menge der Polyethylenglykole, Polyethylenglykole mit einer relativen Molekülmasse zwischen 3 500 und 5 000 aufweisen. Als Bindemittel können jedoch auch Polyethylenglykole eingesetzt werden, welche an sich bei Raumtemperatur und einem Druck von 1 bar in flüssigem Stand vorliegen; hier ist vor allem von Polyethylenglykol mit einer relativen Molekülmasse von 200, 400 und 600 die Rede. Allerdings sollten diese an sich flüssigen Polyethylenglykole nur in einer Mischung mit mindestens einem weiteren Bindemittel eingesetzt werden, wobei diese Mischung wieder den erfindungsgemäßen Anforderungen genügen muss, also einen Schmelzpunkt bzw. Erweichungspunkt von mindestens oberhalb 45 °C aufweisen muss. Ebenso eignen sich als Bindemittel niedermolekulare Polyvinylpyrrolidone und Derivate von diesen mit relativen Molekülmassen bis maximal 30 000. Bevorzugt sind hierbei relative Molekülmassenbereiche zwischen 3 000 und 30 000, beispielsweise um 10 000. Polyvinylpyrrolidone werden vorzugsweise nicht als alleinige Bindemittel, sondern in Kombination mit anderen, insbesondere in Kombination mit Polyethylenglykolen, eingesetzt.

Das verdichtete Gut weist direkt nach dem Austritt aus dem Herstellungsapparat vorzugsweise Temperaturen nicht oberhalb von 90 °C auf, wobei Temperaturen zwischen 35 und 85 °C besonders bevorzugt sind. Es hat sich herausgestellt, dass Austrittstemperaturen - vor allem im Extrusionsverfahren - von 40 bis 80 °C, beispielsweise bis 70 °C, besonders vorteilhaft sind.

### • Extrusion

In einer bevorzugten Ausführungsform wird das erfindungsgemäße Waschmittel mittels einer Extrusion hergestellt. Dabei wird ein festes Vorgemisch unter Druck strangförmig verpresst und der Strang nach Austritt aus der Lochform mittels einer Schneidevorrichtung auf die vorbestimmbare Granulatdimension zugeschnitten. Das homogene und feste Vorgemisch enthält ein Plastifizier- und/oder Gleitmittel, welches bewirkt, dass das Vorgemisch unter dem Druck bzw. unter dem Eintrag spezifischer Arbeit plastisch erweicht und extrudierbar wird. Bevorzugte Plastifizier- und/oder Gleitmittel sind Tenside und/oder Polymere. Zur Erläuterung des eigentlichen Extrusionsverfahrens wird hiermit ausdrücklich auf die obengenannten Patente und Patentanmeldungen verwiesen. Vorzugsweise wird dabei das Vorgemisch vorzugsweise einem Planetwalzenextruder oder einem 2-Wellen-Extruder bzw. 2-Schnecken-Extruder mit gleichlaufender oder gegenlaufender Schneckenführung zugeführt, dessen Gehäuse und dessen Extruder-Granulierkopf auf die vorbestimmte Extrudiertemperatur aufgeheizt sein können. Unter der Schereinwirkung der Extruderschnecken wird das Vorgemisch unter Druck, der vorzugsweise mindestens 25 bar beträgt, bei extrem hohen Durchsätzen in Abhängigkeit von dem eingesetzten Apparat aber auch darunter liegen kann, verdichtet, plastifiziert, in Form feiner Stränge durch die Lochdüsenplatte im Extruderkopf extrudiert und schließlich das Extrudat mittels eines rotierenden Abschlagmessers vorzugsweise zu etwa kugelförmigen bis zylindrischen Granulatkörnern verkleinert. Der Lochdurchmesser der Lochdüsenplatte und die Strangschnittlänge werden dabei auf die gewählte Granulatdimension abgestimmt. So gelingt die Herstellung von Granulaten einer im wesentlichen gleichmäßig vorherbestimmbaren Teilchengröße, wobei im einzelnen die absoluten Teilchengrößen dem beabsichtigten Einsatzzweck angepasst sein können. Im allgemeinen werden Teilchendurchmesser bis höchstens 0,8 cm bevorzugt. Wichtige Ausführungsformen sehen hier die Herstellung von einheitlichen Granulaten im Millimeterbereich, beispielsweise im Bereich von 0,5 bis 5 mm und insbesondere im Bereich von etwa 0,8 bis 3 mm vor.
Das Länge/Durchmesser-Verhältnis der abgeschlagenen primären Granulate liegt dabei vorzugsweise im Bereich von etwa 1 : 1 bis etwa 3 : 1. Weiterhin ist es bevorzugt, das noch plastische Primärgranulat einem weiteren formgebenden Verarbeitungsschritt zuzuführen; dabei werden am Rohextrudat vorliegende Kanten abgerundet, so dass letztlich kugelförmig bis annähernd kugelförmige Extrudatkörner erhalten werden können. Falls gewünscht können in dieser Stufe geringe Mengen an Trockenpulver, beispielsweise Zeolithpulver wie Zeolith NaA-Pulver, mitverwendet werden. Diese Formgebung kann in marktgängigen Rondiergeräten erfolgen. Dabei ist darauf zu achten, dass in dieser Stufe nur geringe Mengen an Feinkornanteil entstehen. Eine Trocknung, welche in den obengenannten Dokumenten des Standes der Technik als bevorzugte Ausführungsform beschrieben wird, ist anschließend möglich, aber nicht zwingend erforderlich. Es kann gerade bevorzugt sein, nach dem Kompaktierungsschritt keine Trocknung mehr durchzuführen. Alternativ können Extrusionen/Verpressungen auch in Niedrigdruckextrudern, in der Kahl-Presse (Fa. Amandus Kahl) oder im Bextruder der Fa. Bepex durchgeführt werden. Bevorzugt ist die Temperaturführung im Übergangsbereich der Schnecke, des Vorverteilers und der Düsenplatte derart gestaltet, dass die Schmelztemperatur des Bindemittels bzw. die obere Grenze des Schmelzbereichs des Bindemittels zumindest erreicht, vorzugsweise aber überschritten wird. Dabei liegt die Dauer der Temperatureinwirkung im Kompressionsbereich der Extrusion vorzugsweise unterhalb von 2 Minuten und insbesondere in einem Bereich zwischen 30 Sekunden und 1 Minute.

### • Walzenkompaktierung

Die erfindungsgemäßen Waschmittel können auch mittels einer Walzenkompaktierung hergestellt werden. Hierbei wird das Vorgemisch gezielt zwischen zwei glatte oder mit Vertiefungen von definierter Form versehene Walzen eindosiert und zwischen den beiden Walzen unter Druck zu einem blattförmigen Kompaktat, der sogenannten Schülpe, ausgewalzt. Die Walzen üben auf das Vorgemisch einen hohen Liniendruck aus und können je nach Bedarf zusätzlich geheizt bzw. gekühlt werden. Bei der Verwendung von Glattwalzen erhält man glatte, unstrukturierte Schülpenbänder, während durch die Verwendung strukturierter Walzen entsprechend strukturierte Schülpen erzeugt werden können, in denen beispielsweise bestimmte Formen der späteren Waschmittelteilchen vorgegeben werden können. Das Schülpenband wird nachfolgend durch einen Abschlag- und Zerkleinerungsvorgang in kleinere Stücke gebrochen und kann auf diese Weise zu Granulatkörnern verarbeitet werden, die durch weitere an sich bekannte Oberflächenbehandlungsverfahren veredelt, insbesondere in annähernd kugelförmige Gestalt gebracht werden können. Auch bei der Walzenkompaktierung liegt die Temperatur der pressenden Werkzeuge, also der Walzen, bevorzugt bei maximal 150 °C, vorzugsweise bei maximal 100 °C und insbesondere bei maximal 75 °C. Besonders bevorzugte Herstellungsverfahren arbeiten bei der Walzenkompaktierung mit Verfahrenstemperaturen, die 10 °C, insbesondere maximal 5 °C oberhalb der Schmelztemperatur bzw. der oberen Temperaturgrenze des Schmelzbereichs des Bindemittels liegen. Hierbei ist es weiter bevorzugt, dass die Dauer der Temperatureinwirkung im Kompressionsbereich der glatten oder mit Vertiefungen von definierter Form versehenen Walzen maximal 2 Minuten beträgt und insbesondere in einem Bereich zwischen 30 Sekunden und 1 Minute liegt.

### • Pelletierung

Das erfindungsgemäße Waschmittel kann auch mittels einer Pelletierung hergestellt werden. Hierbei wird das Vorgemisch auf eine perforierte Fläche aufgebracht und mittels eines druckgebenden Körpers unter Plastifizierung durch die Löcher gedrückt. Bei üblichen Ausführungsformen von Pelletpressen wird das Vorgemisch unter Druck verdichtet, plastifiziert, mittels einer rotierenden Walze in Form feiner Stränge durch eine perforierte Fläche gedrückt und schließlich mit einer Abschlagvorrichtung zu Granulatkörnern zerkleinert. Hierbei sind die unterschiedlichsten Ausgestaltungen von Druckwalze und perforierter Matrize denkbar. So finden beispielsweise flache perforierte Teller ebenso Anwendung wie konkave oder konvexe Ringmatrizen, durch die das Material mittels einer oder mehrerer Druckwalzen hindurchgepresst wird. Die Pressrollen können bei den Tellergeräten auch konisch geformt sein, in den ringförmigen Geräten können Matrizen und Pressrolle(n) gleichläufigen oder gegenläufigen Drehsinn besitzen. Die in dieser Schrift offenbarte Ringmatrizenpresse besteht aus einer rotierenden, von Presskanälen durchsetzten Ringmatrize und wenigstens einer mit deren Innenfläche in Wirkverbindung stehenden Pressrolle, die das dem Matrizenraum zugeführte Material durch die Presskanäle in einen Materialaustrag presst. Hierbei sind Ringmatrize und Pressrolle gleichsinnig antreibbar, wodurch eine verringerte Scherbelastung und damit geringere Temperaturerhöhung des Vorgemischs realisierbar ist. Selbstverständlich kann aber auch bei der Pelletierung mit heiz- oder kühlbaren Walzen gearbeitet werden, um eine gewünschte Temperatur des Vorgemischs einzustellen. Auch bei der Pelletierung liegt die Temperatur der pressenden Werkzeuge, also der Druckwalzen oder Pressrollen, bevorzugt bei maximal 150 °C, vorzugsweise bei maximal 100 °C und insbesondere bei maximal 75 °C. Besonders bevorzugte Herstellungsverfahren arbeiten bei der Walzenkompaktierung mit Verfahrenstemperaturen, die 10 °C, insbesondere maximal 5 °C oberhalb der Schmelztemperatur bzw. der oberen Temperaturgrenze des Schmelzbereichs des Bindemittels liegen.

### • Tablettierung

Die Herstellung von Formkörpern, vorzugsweise solchen in Tablettenform, erfolgt in der Regel durch Tablettierung bzw. Pressagglomerierung. Die erhaltenen teilchenförmigen Pressagglomerate können entweder direkt als Waschmittel eingesetzt oder zuvor nach üblichen Methoden nachbehandelt und/oder aufbereitet werden. Zu den üblichen Nachbehandlungen zählen beispielsweise Abpuderungen mit feinteiligen Inhaltsstoffen von Wasch- oder Reinigungsmitteln, wodurch das Schüttgewicht im allgemeinen weiter erhöht wird. Eine bevorzugte Nachbehandlung stellt die Verfahrensweise dar, bei der staubförmige oder zumindest feinteilige Inhaltsstoffe (die sogenannten Feinanteile) an die erfindungsgemäß hergestellten teilchenförmigen Verfahrensendprodukte, welche als Kern dienen, angeklebt werden und somit Mittel entstehen, welche diese sogenannten Feinanteile als Außenhülle aufweisen. Vorteilhafterweise geschieht dies wiederum durch eine Schmelzagglomeration. In der bevorzugten Ausführungsform der Erfindung liegen die festen Waschmittel in Tablettenform vor, wobei diese Tabletten insbesondere aus lager- und transporttechnischen Gründen vorzugsweise abgerundete Ecken und Kanten aufweisen.

Die Grundfläche dieser Tabletten kann beispielsweise kreisförmig oder rechteckig sein. Mehrschichtentabletten, insbesondere Tabletten mit 2 oder 3 Schichten, welche auch farblich verschieden sein können, sind vor allem bevorzugt. Blau-weiße oder grün-weiße oder blau-grün-weiße Tabletten sind dabei besonders bevorzugt. Die Tabletten können dabei auch gepresste und ungepresste Anteile enthalten. Formkörper mit besonders vorteilhafter Auflösegeschwindigkeit werden erhalten, wenn die granularen Bestandteile vor dem Verpressen einen Anteil an Teilchen, die einen Durchmesser außerhalb des Bereiches von 0,02 bis 6 mm besitzen, von weniger als 20, vorzugsweise weniger als 10 Gew.-% aufweisen. Bevorzugt ist eine Teilchengrößenverteilung im Bereich von 0,05 bis 2,0 und besonders bevorzugt von 0,2 bis 1,0 mm.

### Beispiele

### Beispiel 1

In einer 2-1-Reaktionsapparatur mit Rührer und Destillationsaufsatz wurden 1084 g (0.86 Mol) eines Anlagerungsproduktes von 8 Mol Ethylenoxid an Ricinusöl, 0,45 Natriumborhydrid, 342 g (3.35 Mol) DAPA sowie 28 g einer 33 Gew.-%igen Lösung von Natriummethylat in Methanol vorgelegt. Die Mischung wurde auf 130 °C erhitzt und bei dieser Temperatur 2 h gerührt. Anschließend wurde das überschüssige Amin bei 150 °C und einem verminderten Druck von 40 mmHg bis auf einen Restgehalt von weniger als 0,5 Gew.-% abdestilliert. 74 g (0,16 Mol) des so erhaltenen Amidoamins wurden in eine zweite Reaktionsapparatur überführt, mit 21 g (0,16 Mol) Dimethylsulfat versetzt und anschließend über 4 h bei 65 °C gerührt. Das resultierende Quaternierungsprodukt wurde in praktisch quantitativer Ausbeute als gelblich gefärbte Flüssigkeit erhalten. Der Anteil an nicht-quanterniertem Amidoamin betrug weniger als 5 Gew.-%. Das Produkt wird im weiteren Verlauf als Dehyquart® AU 39 bezeichnet.

### Beispiel 2

In einer 2-1-Reaktionsapparatur mit Rührer und Destillationsaufsatz wurden 1084 g (0.86 Mol) eines Anlagerungsproduktes von 8 Mol Ethylenoxid an Ricinusöl, 0,45 Natriumborhydrid, 342 g (3.35 Mol) DAPA sowie 28 g einer 33 Gew.-%igen Lösung von Natriummethylat in Methanol vorgelegt. Die Mischung wurde auf 130 °C erhitzt und bei dieser Temperatur 2 h gerührt. Anschließend wurde das überschüssige Amin bei 150 °C und einem verminderten Druck von 40 mmHg bis auf einen Restgehalt von weniger als 0,5 Gew.-% abdestilliert. 74 g (0,16 Mol) des so erhaltenen Amidoamins wurden in eine zweite Reaktionsappartur überführt, mit 18 g (0,14 Mol) Dimethylsulfat versetzt und anschließend über 4 h bei 65 °C gerührt. Das resultierende Quaternierungsprodukt wurde in praktisch quantitativer Ausbeute als gelblich gefärbte Flüssigkeit erhalten. Der Anteil an nicht-quanterniertem Amidoamin betrug etwa 10 Gew.-%.

### Stabilität von Haarpflegeemulsionen

Verschiedene siliconöl- und wirkstoffhaltige Emulsionen wurden auf ihre Stabilität untersucht und dabei die folgenden Kationtenside eingesetzt:
A1 methylquaterniertes Ricinolfettsäure+8EO-dimethylaminopropylamin-Methosulfat
A2 methylquaterniertes Kokosfettsäuredimethylaminopropylamin-Methosulfat;
A3 methylquaterniertes Kokosfettsäureamidoethylamin-Methosulfat.

Angegeben ist zudem jeweilss der Anteil an nicht-quaternierten Anteilen, die durch unterschiedliche Einsatzmengen an Alkylierungsmittel in der Quaternierung eingestellt wurden. Die Ergebnisse der Stabilitätsuntersuchungen sind in Tabelle 1 zusammengefasst. Dabei bedeutet (+++) = keine Veränderung, (++) = leichte Trübung, (+) = geringfügige Ausscheidungen, (-) = deutliche Ausscheidungen und (--) vollständige Separierung. Die Beispiele 1 bis 6 sind erfindungsgemäß, die Beispiele V1 bis V6 dienen zum Vergleich.

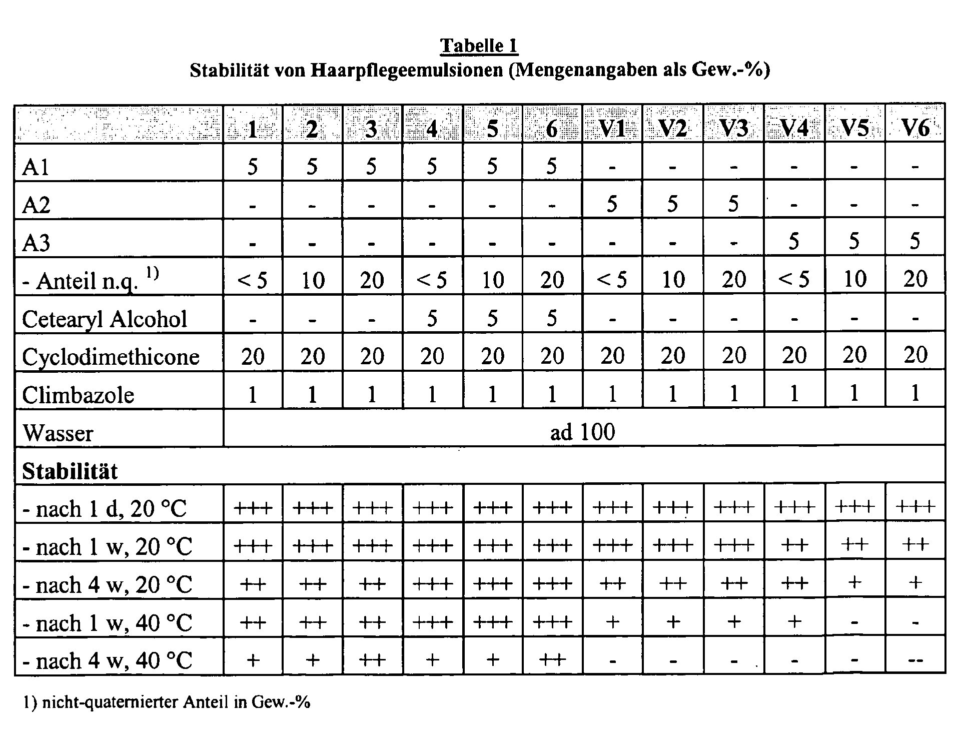

### Avivageleistung und Transparenz wässriger Kationstensidlösungen

Durch Verdünnen mit Wasser wurden 5, 15 und 25 Gew.-%ige Lösungen der drei Kationtenside A1, A2 und A3 aus der vorangegangenen Testserie hergestellt. Die Zubereitungen wurden zum einen unmittelbar nach der Herstellung sowie nach einer Lagerung von 4 Wochen bei 40 °C beurteilt. Des weiteren wurde Frotteegewebe bei 60 °C gewaschen (Waschmaschine : Miele; Waschmittel : Weißer Riese (Henkel), Einsatzmenge 25 g/5 1 Waschflotte, 12 °dH) und der Flotte jeweils 25 ml der 15 Gew.-%igen Kationtensidlösungen zugegeben. Anschließend wurde die Frotteestoffe von einem Panel bestehend aus 10 erfahrenen Prüfern subjektiv auf ihren Weichgriff überprüft. Dabei bedeutet (1) = sehr weich, (2) = weich, (3) = eher hart, (4) hart. Die Ergebnisse sind in Tabelle 2 zusammengefasst; die Ergebnisse zum Weichgriff stellen die Mittelwerte dar. Die Beispiele 7 bis 9 sind erfindungsgemäß, die Beispiele V7 bis V 12 dienen zum Vergleich.

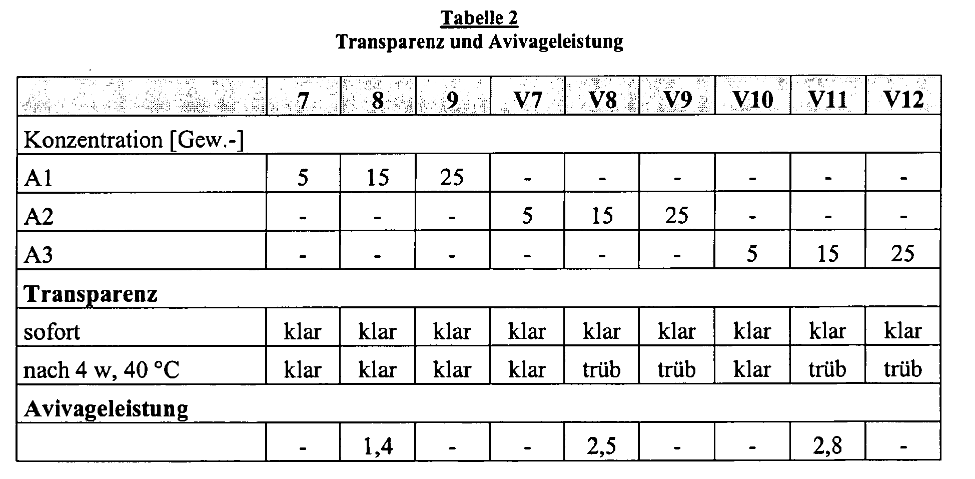

Die nachfolgenden Tabellen 3 und 4 erhalten eine Reihe von Formulierungsbeispielen.

**Tabelle 3 Beispiele für kosmetische Zubereitungen (Wasser, Konservierungsmittel ad 100 Gew.-%)**

| **Zusammensetzung (INCI)** | **1** | **2** | **3** | **4** | **5** | **6** | **7** | **8** | **9** | **10** |
|---|---|---|---|---|---|---|---|---|---|---|
| **Texapon® NSO** Sodium Laureth Sulfate | - | - | - | - | - | - | 38,0 | 38,0 | 25,0 | - |
| **Texapon® SB 3** Disodium Laureth Sulfosuccinate | - | - | - | - | - | - | - | - | 10,0 | - |
| **Plantacare® 818** Coco Glucosides | - | - | - | - | - | - | 7,0 | 7,0 | 6,0 | - |
| **Plantacare® PS 10** Sodium Laureth Sulfate (and) Coco Glucosides | - | - | - | - | - | - | - | - | - | 16,0 |
| **Dehyton® PK 45** Cocamidopropyl Betaine | - | - | - | - | - | - | - | - | 10,0 | - |
| **Dehyquart**® **AU 39** PEG8 Ricinoylamidoethytrimethylammonium Methosulfate | 2,0 | 2,0 | 2,0 | 2,0 | 4,0 | 4,0 | 1,0 | 1,0 | 1,0 | 1,0 |
| **Dehyquart L® 80** Dicocoylmethylethoxymonium Methosulfate (and) Propylenglycol | 1,2 | 1,2 | 1,2 | 1,2 | 0,6 | 0,6 | - | - | - | - |
| **Eumulgin**® **B2** Ceteareth-20 | 0,8 | 0,8 | - | 0,8 | - | 1,0 | - | - | - | - |
| **Eumulgin® VL 75** Lauryl Glucoside (and) Polyglyceryl-2 Polyhydroxystearate (and) Glycerin | - | - | 0,8 | - | 0.8 | - | - | - | - | - |
| **Lanette® O** Cetearyl Alcohol | 2,5 | 2,5 | 2,5 | 2,5 | 3,0 | 2,5 | - | - | - | - |
| **Cutina® GMS** Glyceryl Stearate | 0,5 | 0,5 | 0,5 | 0,5 | 0,5 | 1,0 | - | - | - | - |
| **Cetiol® HE** PEG-7 Glyceryl Cocoate | 1,0 | - | - | - | - | - | - | - | 1,0 | |
| **Cetiol® PGL** Hexyldecanol (and) Hexyldecyl Laurate | - | 1,0 | - | - | 1,0 | - | - | - | - | - |
| **Cetiol® V** Decyl Oleate | - | - | - | 1,0 | - | - | - | - | - | - |
| **Eutanol**® **G** Octyldodecanol | - | - | 1,0 | - | - | 1,0 | - | - | - | - |
| **Nutrilan® Keratin W** Hydrolyzed Keratin | - | - | - | 2,0 | - | - | - | - | - | - |
| **Lamesoft® LMG** Glyceryl Laurate (and) Potassium Cocoyl Hydrolyzed Collagen | - | - | - | - | - | - | 3,0 | 2,0 | 4,0 | - |
| **Euperlan® PK 3000 AM** Glycol Distearate (and) Laureth-4 (and) Cocamidopropyl Betaine | - | - | - | - | - | - | - | 3,0 | 5,0 | 5,0 |
| **Generol® 122 N** Soja Sterol | - | - | - | - | 1,0 | 1,0 | - | - | - | - |
| **Retinol-Nanokapseln gem. Bsp. 3** | 1,0 | 1,0 | 1,0 | 1,0 | 1,0 | 1,0 | 1,0 | 1,0 | 1,0 | 1,0 |
| **Hydagen® CMF** Chitosan | 1,0 | 1,0 | 1,0 | 1,0 | 1,0 | 1,0 | 1,0 | 1,0 | 1,0 | 1,0 |
| **Copherol® 1250** Tocopherol Acetate | - | - | 0,1 | 0,1 | - | - | - | - | - | - |
| **Arlypon® F** Laureth-2 | - | - | - | - | - | - | 3,0 | 3,0 | 1,0 | - |
| **Sodium Chloride** | - | - | - | - | - | - | - | 1,5 | - | 1,5 |

| | | | | | | | | | | |
|---|---|---|---|---|---|---|---|---|---|---|
| (1-4) Haarspülung, (5-6) Haarkur, (7-8) Duschbad, (9) Duschgel, (10) Waschlotion | | | | | | | | | | |

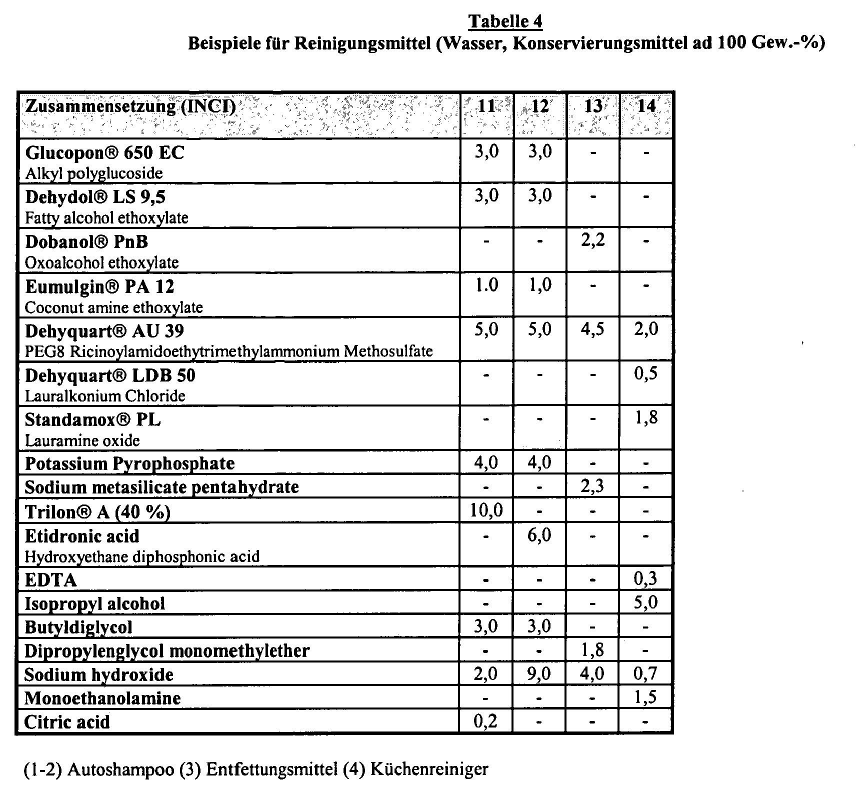

## Patentansprüche

1. Kationische Tenside der Formel (I) 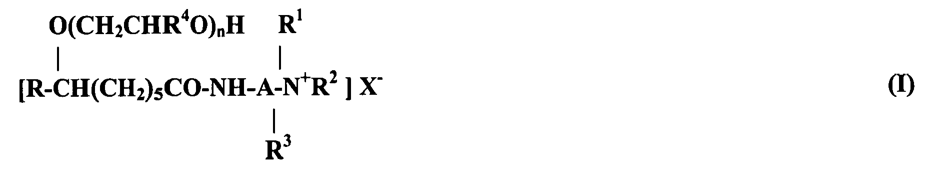 in der R für einen Alkyl- oder Alkenylrest mit 11 Kohlenstoffatomen, R¹ und R² für Wasserstoff oder Alkylreste mit 1 bis 4 Kohlenstoffatomen, R³ für einen Alkylrest mit 1 bis 4 Kohlenstoffatomen, R⁴ für Wasserstoff oder eine Methylgruppe, A für eine lineare oder verzweigte Alkylengruppe mit 2 bis 6 Kohlenstoffatomen, n für Zahlen von 1 bis 25 und X für Halogen oder Alkylsulfat steht.

2. Kationische Tenside nach Anspruch 1, **dadurch gekennzeichnet, dass** sich die quaternierten Amidoamine der allgemeinen Formel (I) von ethoxyliertem Ricinusöl, ethoxlierter Ricinolsäure oder ethoxylierter 12-Hydroxystearinsäure ableiten.

3. Kationische Tenside nach den Ansprüchen 1 und/oder 2, **dadurch gekennzeichnet, dass** R¹, R² und R³ für Methylreste stehen.

4. Kationische Tenside nach mindestens einem der Ansprüche 1 bis 3, **dadurch gekennzeichnet, dass** A für einen Propylenrest steht.

5. Kationische Tenside nach mindestens einem der Ansprüche 1 bis 4, **dadurch gekennzeichnet, dass** n für Zahlen von 5 bis 10 steht.

6. Kationische Tenside nach mindestens einem der Ansprüche 1 bis 5, **dadurch gekennzeichnet, dass** X für Chlorid oder Methylsulfat steht.

7. Verfahren zur Herstellung von kationischen Tensiden der Formel (I) 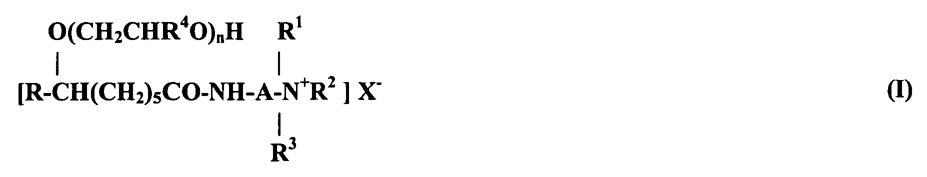 in der R für einen Alkyl- oder Alkenylrest mit 11 Kohlenstoffatomen, R¹ und R² für Wasserstoff oder Alkylreste mit 1 bis 4 Kohlenstoffatomen, R³ für einen Alkylrest mit 1 bis 4 Kohlenstoffatomen, R⁴ für Wasserstoff oder eine Methylgruppe, A für eine lineare oder verzweigte Alkylengruppe mit 2 bis 6 Kohlenstoffatomen, n für Zahlen von 1 bis 25 und X für Halogen oder Alkylsulfat steht, bei dem man (a) Anlagerungsprodukte von durchschnittlich 1 bis 25 Mol Ethylen- und/oder Propylenoxid an eine Hydroxycarbonsäure oder deren Glycerid mit einem Diamin der Formel (II) umsetzt, 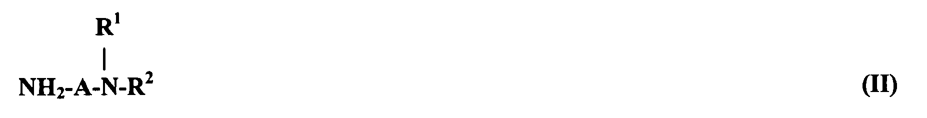 in der R¹, R² und A die oben genannten Bedeutungen haben, und (b) anschließend die so erhaltenen alkoxylierten Hydroxyfettsäureamidoamine mit einem Alkylhalogenid oder Dialkylsulfat quaterniert.

8. Verfahren nach Anspruch 7, **dadurch gekennzeichnet, dass** man Anlagerungsprodukte von durchschnittlich 1 bis 25 Mol Ethylenoxid an Ricinusöl oder Ricinolsäure mit Dimethylaminopropylamin (DAPA) umsetzt und das resultierende ethoxylierte Amidoamin anschließend mit Dimethylsulfat quaterniert.

9. Verwendung von Kationtensiden nach Anspruch 1 zur Herstellung von kosmetischen und/oder pharmazeutischen Zubereitungen.

10. Verwendung von Kationtensiden nach Anspruch 1 zur Herstellung von Wasch-, Spül-, Reinigungs- und Avivagemitteln.

11. Verwendung nach Anspruch 10, **dadurch gekennzeichnet, dass** das Mittel ein Entfettungsmittel, ein Allzweckreiniger, ein Küchenreiniger, ein Ofenreiniger, ein Mittel zur Reinigung harter Oberflächen oder eine Teppichreiniger ist.

12. Verwendung nach Anspruch 10, **dadurch gekennzeichnet, dass** die Mittel zur Reinigung von Metallteilen, speziell Fahrzeugen und Fahrzeugteilen dienen.

13. Verwendung nach Anspruch 10, **dadurch gekennzeichnet, dass** die Mittel zur Korrosionsinhibierung dienen.

14. Verwendung nach Anspruch 10, **dadurch gekennzeichnet, dass** die Mittel zur Verminderung der statischen Aufladung von harten Oberflächen, speziell von Möbeln, Farben, Fenstern, Monitoren oder Plastikgegenständen dienen.

15. Verwendung von Kationtensiden nach Anspruch 1 zur Ausrüstung von Fasern, Garnen und Textilien.
